# EUROPEAN PATENT APPLICATION

(11) **EP 1 477 572 A2**
(43) Date of publication of application: **17.11.2004**
(21) Application number: 04016101.0
(22) Date of filing: 04.10.1996
(51) Int. Cl.: C12Q 1/68, G01N 27/447

(54) **Methods and kit for hybridization analysis using peptide nucleic acid probes**

(30) Priority: 06.10.1995 US 4953 P
(62) Divisional of application: 96934037.1
(71) Applicant: PERSEPTIVE BIOSYSTEMS, INC., Framingham, MA 01710 (US)
(72) Inventor: Fuchs, Martin, Uxbridge, MA 01569 (US); Egholm, Michael, Lexington, MA 02173 (US); O' Keefe, Heather, Lexington, MA 02173 (US); Yao, Xian W., Framingham, MA 01701 (US)
(74) Representative: Grund, Martin, Dr.

(57) **Abstract**

A method, composition and apparatus for the hybridization and separation of molecules having a desired target sequence in a sample by contacting a sample of single stranded nucleic acids with a detectable PNA probe having a sequence complementary to the target sequence so that the target sequence, if present, will hybridize with the detectable probe to form a detectable duplex, and then separating the duplex in a denaturing medium from unbound sample components by electrophoresis. The invention also relates to methods, compositions and apparatus for the hybridization and separation of molecules having a desired target sequence in a mixed sample of single stranded nucleic acids and their complementary strands by contacting the sample with a detectable PNA probe.

## Description

### Field of the Invention

The present invention relates generally to the field of hybridization analysis and more specifically to hybridization analysis using peptide nucleic acids as probes for DNA or RNA target sequences.

### Background of the Invention

Electrophoresis, a well established technique for the separation and analysis of mixtures, involves the migration of molecules in an electric field and their separation based on differences in mobility. Many different forms of electrophoresis have been developed including free zone electrophoresis, gel electrophoresis, isoelectric focusing and isotachophoresis. Traditionally, electrophoresis has been performed in gels cast from polyacrylamide or agarose in the form of slabs or rods. Slab gels have been widely used for the separation and analysis of polynucleotides such as DNA or RNA

Electrophoresis may be combined with other analytical techniques in order to determine the sequence of nuclcleotides in polynucleotide target of interest. In a technique known as Southern blotting, a DNA sample is analyzed for a "target" nucleotide sequence of interest. The DNA sample is denatured, typically by heating, and the denatured single stranded DNA is electrophoretically separated in a gel slab. After electrophoretic separation, the DNA is fragmented and transferred, or blotted, from the gel to a membrane, such as a nitrocellulose membrane. The single stranded fragmented DNA is then probed with a labeled DNA or RNA oligomer having a sequence complementary to the target nucleotide sequence of interest. The DNA or RNA oligomer, or probe which is complementary to the target sequence will hybridize to the target sequence, if present. Any unbound DNA fragments and excess probe are then removed by stringent washing. The label on the specifically bound oligonucleotide probe remains on the membrane after washing and is detected. The presence or absence of the target sequence and its size can be easily determined. A similar technique, referred to as northern blotting, is used to analyze RNA samples with either RNA or DNA probes.

Reliable and reproducible results often require experimental optimization for each individual system. In addition, the most critical step is often the stringent washing step which is required to effectively desorb any nonspecifically bound probe (which can lead to an increase in background counts) without removing the specifically bound probe necessary for making a measurement. Further, blotting techniques are complex procedures which require that the target nucleotide sequence of interest be denatured prior to electrophoresis and that the probe not be added until the blotting has taken place. Thus, the blotting techniques described above are complex, time consuming and labor intensive with each overall procedure requiring from 4 hours to several days.

Peptide Nucleic Acids or PNAs have been determined to be useful probe substitutes in conventional northern and Southern blot analysis. See Pluskal, M. et al., Peptide Nucleic Acid Probes and Their Application in DNA and RNA Blot Hybridization Analysis, *The FASEB Journal.* Poster #35 ( 1994). PNAs are synthetic oligoamides comprised of repeating units of amino acid, to which the nucleobases adenine, cytosine, guanine, thymine and uracil are attached. See Egholm et al., *Nature* (1993) 365, 566-568; Oerum et al. *Nucl. Acids Res.* (1993) 23 5332-36; *Practical PNA: Identifying Point Mutations by PNA Directed PCR Clamping* (1995) PerSeptive Biosystems Vol. 1, Issue 1. PNA synthons and oligomers are commercially available from PerSeptive Biosystems, Inc., Framingham, MA, and can be made by methods known in the art. See for example PCT applications PCT/EP92101219, PCT/EP92/01220, PCT/US92/10921).

In many applications, PNA probes are preferred to nucleic acid probes because, unlike nucleic acid/nucleic acid duplexes which are destabilized under conditions of low salt, PNA/nucleic acid duplexes are formed and remain stable under conditions of very low salt. See Egholm et al., *Nature* (1993) 365, 566-568; *Practical PNA: PNA Oligomers as Hybridization Probes* (1995) PerSeptive Biosystems, Vol. 1, Issue 2. Additionally, because PNA/DNA complexes have a higher thermal melting point than the analogous nucleic acid/nucleic acid complexes, use of PNA probes can improve the reproducibility of blotting assays. See Pluskal M., et al. However, even with PNA probes, the assays remain complex, both time consuming and labor intensive.

The electroseparation of unlabeled PNA/DNA oligonucleotide complexes in a gel filled capillary has been described. Rose, D. J., *Anal. Chem.* (1993) 65: 3545-3549. Although this technique may be advantageous because of the stability of the PNA/DNA hybrid, the PNA/DNA hybrids are formed in a non-competing environment where complementary DNA strands were not available to compete with the PNA in the formation of the PNA/DNA complex. This experiment did not show if it is possible to form PNA/DNA complexes starting with double stranded DNA

Such double stranded nucleic acid samples pose specific problems when performing hybridization analysis. Because each strand of a double stranded sample has a very high affinity for its complementary strand, even under conditions of low salt, oligonucleotide probes cannot efficiently compete for binding in the presence of a DNA strand which is complementary to the target DNA strand of interest. Thus, the analysis of a double stranded DNA typically requires the probing of a single stranded nucleic acid sample under conditions where the complementary strand is not present, or is not available for competition with the probe. For example, in traditional blotting techniques, one blots the fragments from a gel to a membrane to immobilize competing complementary fragments. Thus such techniques add additional complexity and time to the analysis of DNA sequences.

Since double stranded DNA is the most common form of nucleic acid in a genomic sample, the available techniques are therefore not entirely satisfactory. There exists a need for rapid, reproducible, easy and cost effective method for the detection and analysis of nucleic acids. The present invention addresses these needs.

### Summary of the Invention

Accordingly, the present invention is directed to a method, compositions, an apparatus, and kits for the separation and detection of polynucleotide samples having a target sequence of interest and that substantially obviate one or more of the problems due to the limitations and disadvantages of the prior art.

In one embodiment, the invention relates to a method for the separation of the components of a mixed sample solution of single stranded polynucleotide sequences and their complementary strands, and for detecting therein a selected target sequence. To practice the method, one first denatures a double stranded polynucleotide, typically by heating, into complementary single stranded polynucleotide pairs. Next the sample of single stranded polynucleotide pairs are mixed with a PNA probe having a sequence complementary to at least a portion of the selected target sequence to thereby hybridize the single stranded polynucleotide sequence with PNA to form a detectable PNA/polynucleotide duplex. The various polynucleotide and polynucleotide/PNA species in the sample are then separated electrophoetically and the detectable PNA/ polynucleotide duplex is detected. The PNA probe may be labeled with a detectable moiety, such as enzymes, fluorophores, biotin, chromophores, radioisotopes, colored particles, electrochemical or chemiluminescent moieties. In various embodiments, the various species are separated in a sieving medium, electrophoretically, or, more specifically, by capillary electrophoresis. The sieving medium, if used, may be selected from the group consisting of polyacrylamide, agarose, polyethylene oxide, polyvinyl pyrolidine and methylcellulose. In other embodiments, no sieving medium is used.

In other embodiments, the claimed invention relates to an apparatus for the detection in a sample of a polynucleotide comprising a selected target sequence. The apparatus of the invention has a sample injection zone, an electrophoretic sieving medium in communication with said injection zone, and, a PNA probe, disposed so as to mix with a sample introduced to the injection zone. The PNA probe has a sequence complementary to at least a portion of the selected target sequence, and hybridizes with the target sequence, if present, to form a detectable complex. The probe may be labeled, and any appropriate sieving medium may be used. In a preferred embodiment, the apparatus of the present invention comprises at least one capillary or channel. In another embodiment, the apparatus has a plurality of channels. In yet another preferred embodiment, the apparatus is a multichannel microchip.

In yet other embodiments, the claimed invention relates to the above methods, compositions and apparatus wherein at least two PNA probes are labeled, each of which hybridizes with a different target sequence, if present, to form a detectable complex. The target sequences may be present on the same DNA segment or separate DNA segments, and may originate from different loci on the same gene or from different genes.

It is to be understood that both the foregoing general description and the following detailed description are exemplary and explanatory and are intended to provide further explanation of the invention as claimed. The accompanying drawings are included to provide a further understanding of the invention and are incorporated in and constitute a part of this specification, illustrate several embodiments of the invention, and together with the description serve to explain the principles of the invention.

### Description of the Drawings

Figure 1 is a flow diagram of traditional Southern Blotting techniques with a flow diagram of an embodiment of the "no-blot" method of the invention.
Figures 2A and 2B depict, on two segments of an agarose gel, the results of DNA/DNA and DNA/PNA hybridization experiments at various temperatures and concentrations developed by chemiluminescence.
Figures 3A, 3B and 3C show the results of a hybridization experiment between two restriction digests of pBR322 with a labeled PNA probe separated on three different electrophoretic gel systems: non denaturing 4-20% acrylamide (3A); 7 M urea 6% acrylamide (3B); and 1% agarose (3C).
Figure 4 shows the effect of temperature in a hybridization experiment between double stranded DNA pBR322 and labeled PNA probe.
Figures 4A and 4B show the results of pre-gel hybridization assays for the Cystic Fibrosis gene W1282 provided by an unaffected individual (H), lanes 1, 2, and 5 and a carrier (C), lanes 3 and 4, with a labeled PNA probe for the wild type (WT) gene, lanes 1 and 3, or the mutant (M) gene, lanes 2 and 4, and an excess of mutant probe (lane 5)on a 1.5%TBE agarose gel: DNA.
Figure 5 is a plot comparing the stability of complementary (curve A: ◆), one-base mismatch (curve B: **■)** and two-base mismatch (curve C: **•)** PNA/DNA duplexes in a denaturing medium at various temperatures.
Figure 6 is a plot comparing the stability of complementary (curve A: ◆) and two-base mismatch (curve B: **■)** PNA/DNA duplexes at various temperatures.
Figure 7 is an electropherogram of capillary gel electrophoresis of DNA fragments from the BstNI and MspI digestion of pBR322.
Figure 8 is a size calibration curve for the size separation of digestion fragments of pBR322 by capillary electrophoresis.
Figure 9 is an electropherogram of an electrophoretic separation of the components of a hybridization assay containing a labeled PNA probe and the fragments of a BstN I digestion of pBR322.
Figure 10 is an electropherogram of an electrophoretic separation of the components of a hybridization assay containing a labeled PNA probe and the fragments of a Msp I digestion of pBR322.
Figure 11 is an electropherogram of an electrophoretic separation of the components of a hybridization assay containing a labeled PNA probe and the fragments of both the BstN I digestion of pBR322 and the Msp I digestion of pBR322.
Figure 12 is a schematic representation sectional of a perspective view in the plane of the capillary of an embodiment of the apparatus of the invention.
Figure 13 is a schematic representation of the sample loading process (panel A) and the eletrophoresis separation process (panel B) with an offset pinched injector.
Figure 14 is a schematic representation of a sectional perspective view in the plane of the capillaries of a multichannel capillary microchip wafer.
Figure 15 is a schematic representation of the integrated apparatus for hybridization analysis using capillary gel electrophoresis.
Figure 16 is an electropherogram of an electrophoretic separation of a hybridization assay containing a labeled PNA probe and the fragments of both the BstN1 and Msp1 digestions of pBR322 using a multichannel microchip.
Figure 17A shows electropherograms of electrophoretic separations of the components of hybridization assays containing a labeled PNA probe for the wild type Cystic Fibrosis F508 gene with DNA samples provided by: (a) an unaffected individual; (b) a carrier; and (c) an affected patient.
Figure 17B shows electropherograms of electrophoretic separations of the components of hybridization assay containing a labeled PNA probe for the mutant Cystic Fibrosis F508 gene (ΔF508) with DNA samples provided by: (a) an unaffected individual; (b) a carrier; and (c) an affected patient.
Figure 18A shows electropherograms of electrophoretic separations of the components of hybridization assays for the wild type Cystic Fibrosis W1282 gene on a wild type DNA samples provided by an unaffected individual: (a) wild type labeled PNA probe; (b) a mutant labeled PNA probe; and (c) control without DNA sample but with mutant probe.
Figure 18B shows electropherograms of electrophoretic separations of the components of hybridization for the mutant Cystic Fibrosis W1282X gene on a DNA samples provided by a healthy carrier: (a) wild type labeled PNA probe; (b) a mutant labeled PNA probe.
Figure 19 is an electropherogram of an electrophoretic separation of the components of a multiplex hybridization assay containing two labeled PNA probes for the wild types cystic fibrosis W1282 and F508 genes.

### Detailed Description of the Invention

The inventors have found that PNA will remain hybridized with a target DNA or RNA sequence under what are normally denaturing conditions for a DNA/DNA, RNA/DNA or RNA/RNA hybrid. Thus the practitioner is capable of simultaneously detecting the presence of a target sequence based upon hybridization with the probe to form a complex, as well as electrophoretically separating sample components on a sieving medium. A "probe" as used herein, refers to a sequence of nucleotide bases complementary to at least a portion of the nucleobases in the target sequence. Previously, it was necessary to render the complementary nucleic acid strand unavailable for binding to its partner, prior to hybridization with a probe so as to prevent competition between the completmentary DNA target and the DNA probe. Thus the hybridization between the target nucleotide sequence and the PNA probe can be performed under conditions in which the DNA/DNA probe would not hybridize. The term "target sequence", as used herein, refers to any sequence of nucleotide bases which the artisan wants to detect, such as a DNA or RNA sequence, and which is capable of hybridizing with a PNA probe. As an added benefit, unlike conventional nucleotide probes, PNAs do not have a charge, and therefore simplify the electrophoretic separation of the hybrid from the excess probe. Assays using these PNA probes are at least as sensitive and discriminating as commonly used oligonucleotide probes, and hybridize more rapidly to their targets. Furthermore, the assays of the invention provide for rapid, one-step hybridization followed by size separation, and therefore are less time consuming, complex and labor intensive than methods known in the art.

The term PNA (peptide nucleic acid), as used herein, refers to a DNA mimic with a neutral polyamide backbone on which the nucleic acid bases are attached in the same manner as they are to the phosphate backbone of DNA. 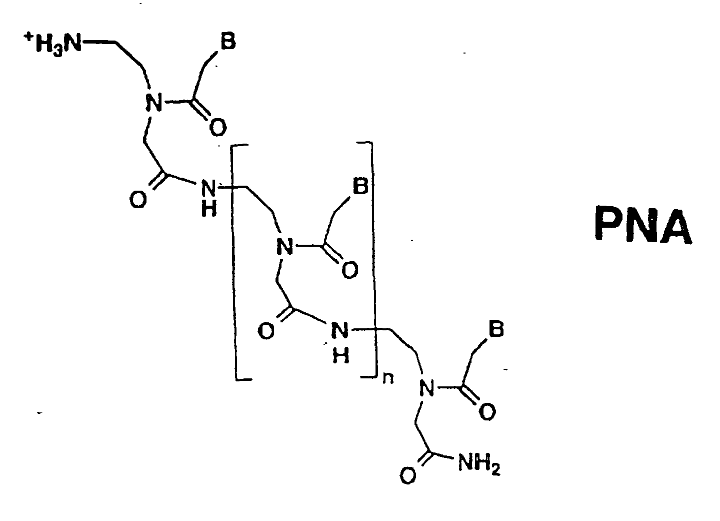 PNAs can be synthesized routinely by standard peptide chemistry and can be labeled with biotin or various other labels as either part of the solid phase synthesis, or following cleavage from the resin. See, e.g., Chollet et al., *Nucleic Acids Research,* Vol. 13, No.5, pp. 1529-1541 (1985). Methods of making PNA from monomer synthons are known to the art.

"Detectable moieties", as used herein, are moieties suitable for use in the claimed invention, including, but not limited to: enzymes, fluorophores, biotin, chromophores, radioisotopes, electrochemical moieties and chemiluminescent moieties. Such moieties can be readily conjugated with the probe using art-recognized techniques.

To fully appreciate all aspects of the claimed invention, it should be observed that the applicants have discovered two important properties of PNA/nucleic acid duplexes which lead to the compositions, kits and methods of this invention. First, the applicants have surprisingly observed that when a labeled PNA probe having a sequence complementary to a target sequence is added to a double stranded nucleic acid sample which has been denatured, the PNA probe will rapidly hybridize with the target sequence to thereby form a duplex in the presence of the complementary nucleic acid strand. Because the longer complementary nucleic acid strand will compete with the much shorter PNA probe for binding, even under conditions of low salt, it is surprising that the PNA probe is not easily or rapidly displaced to thereby form the more stable double stranded nucleic acid starting material. This is very surprising for samples containing long nucleic acid fragments such as samples having double stranded sections of greater than 50 nucleotide subunits in length.

Second, the PNA/nucleic acid duplex is stable under denaturing conditions in which DNA secondary structure is disrupted. Therefore, it is possible to separate components of a sample under denaturing conditions, thereby preventing secondary structure formation and binding of complementary fragments. This is not possible using traditional DNA or RNA probes since the denaturing conditions would affect not only desirable fragmentation, i.e. secondary structure formation, but would also disrupt the desired hybridization with probe. The presence of denaturing reagents, such as formamide, will lower the temperature needed to achieve single base pair discrimination of probe sequences for targets. Thus, the annealing step of hybridization assays can therefore be performed at or about ambient temperature. Consequently, the hybridization assays of this invention can be much more discriminating than the assays of the related art.

In the prior art applications, hybridization of the probe is not performed prior to the separation of the two independent strands of DNA. In traditional techniques labeled probes migrate in the electric field and are used in large excess. Therefore, excess probe appears as a very intense signal which can interfere with acurate detection of some targets. Additionally, since the polynucleotide/probe complex is easily dissociated, little or no signal may be available for detection, depending on the degree of dissociation. If the separation medium included a denaturing reagent, all complex would be rapidly dissociated and none would be detected.

Unlike labeled DNA and RNA probes, the labeled PNA probes of the invention can remain hybridized to target sequences in nucleotide samples even in the presence of a complementary nucleotide strand. The PNA/nucleic acid complex formed is very stable under conditions of low salt, and sufficiently stable in denaturing reagents to allow separation and detection. Additionally, since the PNAs are uncharged, and do not migrate substantially in electrophoretic separation, a better separation of components is obtained.

The single stranded nucleic acids can be obtained from any natural or synthetic source. For example, any sample of DNA or RNA in which one wants to detect the desired sequence may be fragmented and denatured by any methods known in the art. Thus, for example, in certain diagnostic applications one may detect the presence of a certain sequence by digesting at least a portion of the human genome, denaturing it, and using it as a sample in the methods of the invention.

In one embodiment, one can denature double stranded nucleotide fragments and hybridize a PNA probe to one of them before the nucleotide fragments reanneal, by using a low ionic strength buffer, and by raising the temperature to about 65 or greater for approximately 5 minutes. In this embodiment the sample comprising the denatured nucleotide fragments and PNA probe, is now loaded directly on the gel and placed under an electric field. A hybridized complex comprising a PNA probe and a complementary DNA or RNA sequence will exhibit an electrophoretic mobility different from that of the unbound probe and different from that of the single stranded nucleotides. Because the PNA probe is neutral, unhybridized probes will not migrate in the gel under an electric field. In some cases the label attached to the probe may add some charge to the probe, however, the electrophoretic mobility of the labeled probe is generally low and sufficiently different from that of the hybridized PNA/nucleotide duplex to permit a rapid, complete separation. Thus, only the single stranded nucleic acid fragments and hybridized complexes will enter and migrate in the gel. Fragments containing the target sequences can therefore be detected by detecting the label on the probe which has hybridized with the fragment. Note that with this technique no additional steps are required to separate the complementary nucleotide strands to prevent competition with the PNA probe.

In brief overview, the methods of the invention may be practiced by first designing a PNA probe with a sequence complementary to, and capable of hybridizing with, at least a portion of a target sequence of interest. A target sequence is any known sequence of nucleotide bases which one wants to detect in a sample. Target sequences may, in various embodiments, be sequences which code for a gene of interest. Examples of target sequences include, but are not limited to, promoter sequences, loci, sequences specific to pathogens, sequences specific to oncogenes, sequences specific to genetically altered plants and organisms, sequences specific to genetic defects, sequences amplified by methods such as PCR, and sequences coding for common structural motifs such as zincfingers, etc.

In more detail, PNAs can be synthesized by methods which are analogous to those used for the synthesis of peptides. Methods of making PNAs from monomer synthons are known in the art. The PNA probes of the invention are preferably labeled with a detectable moiety such that, upon hybridization with a target sequence, a labeled PNA/nucleic acid complex is formed. Fluorescein or biotin labeled PNA probes can be synthetized on an Expedite Nucleic Acid Synthesis System from PerSeptive Biosystems (Framingham, MA, USA). Spacers units of 8-amino-3,6-dioxaoctanoic acid (-oo-) are added to the resin-bound PNAs before reacting activated esters of biotin (Bio) or fluorescein (Flu) such as dimethoxytritylbiotin ester of 1-(4'-nitrophenyl)pyrazolin-5-one (DMTr-bio-HPP) or 5,6-carboxyfluorescein-N-hydroxysuccinimide. The labeled PNAs are then cleaved from the resin and protecting groups removed to give the labeled PNA probes used in the present invention. Methods for labeling PNAs with fluorescent dyes are also known to one skilled in the art.

The PNA probe correspondingto the targhet sequence of interest is then introduced into a sample of polynucleotides to be tested. The sample may comprise DNA or RNA and may be double stranded or single stranded. If the sample is originally double stranded, the sample, when denatured prior to the addition of the PNA probe, will comprise a solution of single stranded polynucleotides and their complementary strands. The probe will compete with the complementary strand of DNA and preferentially bind with at least a portion of the target sequence, if present, to form a detectable duplex.

It is believed that, in part, the differences in the performance of PNA and DNA probes is due to the differences in the on and off rates of the molecule. The faster on-rate of PNA allows shorter hybridization times to be used. The slower off-rate of PNA enables one to discern mismatches more readily, due to the ability of the PNA to hybridize and withstand washing over a broader range of temperatures than can be used with DNA probes. Additionally, the off-rate differences between the DNA and PNA probes become greater as the length of the probe is decreased. In fact, in the prior art oligonucleotides less than 13 residues long could not be used as probes, whereas PNA as short as 10 residues gives ample signal for detection. Thus, according to the present invention, one may design PNA probes based upon as little as four residues of protein sequence information. PNA is therefore useful for a variety of hybridization based DNA/RNA applications, including, but not limited to, direct detection of point mutations, solid-phase affinity capture and improvement of PCR for DNA containing repeat sequences.

The salt independence of PNA binding to DNA and RNA is a particularly attractive feature of the claimed invention. PNA can bind tightly under low salt conditions, for example concentrations of less than about 10mM. Under these conditions, DNA/DNA, DNA/RNA or RNA/RNA duplex formation (i.e. secondary and tertiary structure) is highly disfavored or prevented. Therefore, it may be preferable to mix the nucleic acid sample with the PNA probe under low ionic strength conditions. These conditions will favor the PNA probe/nucleic acid hybridization, which is relatively unaffected by the salt concentration, whereas the nucleic acid/nucleic acid interactions are favored in higher ionic strength conditions. Preferably, the ionic strength of the mixture is in the range of about 1 to about 50 millimolar.

In certain embodiments, it may be preferable to incubate the sample and probe for a period of time, i: e. from about 1 minute to 30 minutes, to afford a more prolonged opportunity for the components to interact and form complex prior to loading on the sieving medium. The skilled artisan will be able to determine when it is appropriate and/or necessary to include the incubating step using routine experimentation.

After mixing the labeled probe with the nucleotide sample, hybridized PNA/nucleic acid duplexes must be separated from the excess labeled probe. Preferably, the duplexes will also be separated from other species in the sample such as the complementary nucleotide strands not having the target sequence, and other unbound components. Numerous separations technologies are known in the art, and are useful in the methods of the invention. For example, species in a sample may be separated by electrophoresis, chromatography, mass spectrometry and other methods. Preferably, the components are separated by electrophoresis with or without a sieving medium. However, it may also be preferable to separate the components on a chromatographic medium, such as, for example, a perfusion chromatography medium, i.e. POROS® available from PerSeptive Biosystems, Inc.

In certain embodiments, a sieving medium is formed into a slab gel comprising polyacrylamide or agarose. As used herein, a sieving medium can be any medium capable of separating species in a sample based upon size. One skilled in the art can easily select an appropriate medium based upon the particular size separation desired. The sieving medium may, for example, be selected from the group consisting of polyacrylamide, agarose, polyethylene oxide, polyvinyl pyrolidine and methylcellulose. The medium may be a solution, or be in liquid or gel form, and may be in a slab or capillary. The electrophoretic velocity of a chemical component is determined by its electrophoretic mobility in an electric field and the electro-osmotic flow. The electrophoretic mobility of the component can be affected by the nature of the electrophoretic medium, e.g., pH, ionic strength and viscosity. An electrophoretic medium can be chosen for physical properties which will selectively impede the electrophoretic mobilities of certain components of the system. For example, the amount of sieving agent added to the medium can alter the molecular drag of the species and, therefore, alter the electrophoretic mobility. In certain embodiments, no sieving medium is needed, the buffer being a sufficient electrophoretic medium for the separation of the components. In other embodiments, the medium may be denaturing. Those skilled in the art will recognize that a medium may be rendered denaturing by any means known in the art, such as, increased temperature or addition of denaturing reagents.

The electric potential required to impart electrophoretic motion is typically applied across the medium by a high voltage source operated at electric field strengths generally ranging from one hundred volts per centimeter to several thousand volts per centimeter. Preferably the electric filed is about 100 to about 500 volts per centimeter. See for example U.S. Patents 4,865,706 and 4,865,707. Because PNAs have a polyamide backbone, they are essentially uncharged. Thus, the separation of the PNA/DNA hybrid from excess probe is relatively easy, since the highly charged hybrid will migrate rapidly in the electric field while the essentially neutral probe will migrate slowly or not at all. As discussed above, a label may add a small charge to the probe, however, does not significantly affect the separation of the PNA/nucleotide complex as would a DNA or RNA probe.

In one embodiment the sieving medium is disposed within an electroseparation channel, and sample and probe added thereto. The electrophoresis can be advantageously carried out by capillary electrophoresis in capillaries or channels, with or without sieving medium. Although the capillaries or channels can be of any size, small cross-sectional area of capillaries or channels allow very high electric fields to be used for high speed and high resolution. A channel is preferably about 0.1 µm to about 1000 µm in depth and about 10 µm to 1000 µm in width. A capillary is preferrable between about 25 µm to 100 µm diameter. Such a format lends itself readily to quantitative detection of the label by fluorescence or UV absorbance. It is well suited for automation. In certain embodiments, a multichannel microchip may be used to allow high throughput by performing multiple assays in parallel. A microchip may contain up to 100 channels.

An electrical potential is then applied to the electrically conductive sieving medium contained within the channel to effectuate migration of the hybridized duplex, and other components of the sample. Many experimental parameters of the claimed invention may be varied, including, but not limited to, electro-osmotic flow, electrophoretic mobility, chemistry of the electrophoretic medium, pH, temperature, ionic strength, viscosity, sample volume, electric potential, length of capillary, nature and amount of sieving medium, detection method etc. These parameters may be optimized for any electroseparation analysis performed. Varying one or more of these parameters allows one of skill in the art using routine experimentation to exploit the invention, and confers versatility on the claimed methods.

Following the step of separation, the claimed method provides for detection of the hybridized complex. Detection can be achieved by methodologies including, but not limited to: absorbance of ultraviolet radiation, absorbance of visible radiation, fluorescence, chemiluminescence, refractive index, Raman spectroscopy, mass spectrometry, electrochemistry and conductivity. In some embodiments, it may be preferable to label the PNA probe with biotin, which has been exploited for the detection or localization of proteins, carbohydrates and nucleic acids. See Chollet et al., *Nucleic Acids Res.,* Vol. 13, No. 5, pp.1529-1541 (1985).

The skilled practitioner may utilize other moieties in combination with the sample sequences to increase the resolution of the separation. For example, charge-modifying moieties may be added to the mixture to increase the force experienced by the PNA/nucleotide complex under the electric field. Briefly such a method for the electroseparation analysis of such a mixture involves the step of electrically separating in a channel a mixture containing (1) a sample; (2) a first binding partner which binds to a first binding site on an analyte; and (3) a second binding partner which binds to a second binding site on the analytes. The first binding partner may comprise a detectable moiety, the second binding partner may comprise a charge-modifying moiety, thus, attributing a different electrophoretic mobility from unbound sample and components.

In certain embodiments, it may be useful to probe for more than one target sequence in a particular sample. Such multidimensional analyses can be performed in various ways. For example, if the separation is based on size, the pattern of fragments detected in such an assay may be unique and therefore the basis of an assay. Similarly, when separating by electrophoresis, the pattern may appear as an electropherogram, and in other embodiments, the pattern may appear as spots or bands on a photographic film. One skilled in the art can easily perform such multidimensional assays by contacting a sample with more than one labeled PNA probe directed to more than one target sequence. The labels on the various probes can be the same or different. Upon detection, the pattern of signals may be analyzed for the target sequences. These multidimensional methods may be useful, for example, in the identification of particular genes, gene polymorphism, paternity testing, or diagnostic applications.

In a preferred embodiment, the labels may comprise different fluorescent labels which are independently detectable. See for example Smith, L.M. et al., *Nucleic Acids Research,* 13:2399-2412 (1985); and Smith, L.M. et al., *Nature,* 321: 674-679 (1986). In certain assays, use of multiple labels may facilitate the absolute identification of whether a target sequence is associated with a particular fragment.

Additionally, multiple samples can be processed in a single separation wherein the probe or probes used to perform the analysis of a single sample all have a common label but the labels differ for different samples. Thus, in one embodiment the labels can be fluorescent labels and the separation can be carried out by electrophoresis. Accordingly, all samples can be pooled and separated in a single separation. All information for all samples is available upon hybridization and can be captured using a detection system which can distinguish the differing labels. Using appropriate data interpretation tools, data for each individual sample can be individually represented and reported when desired.

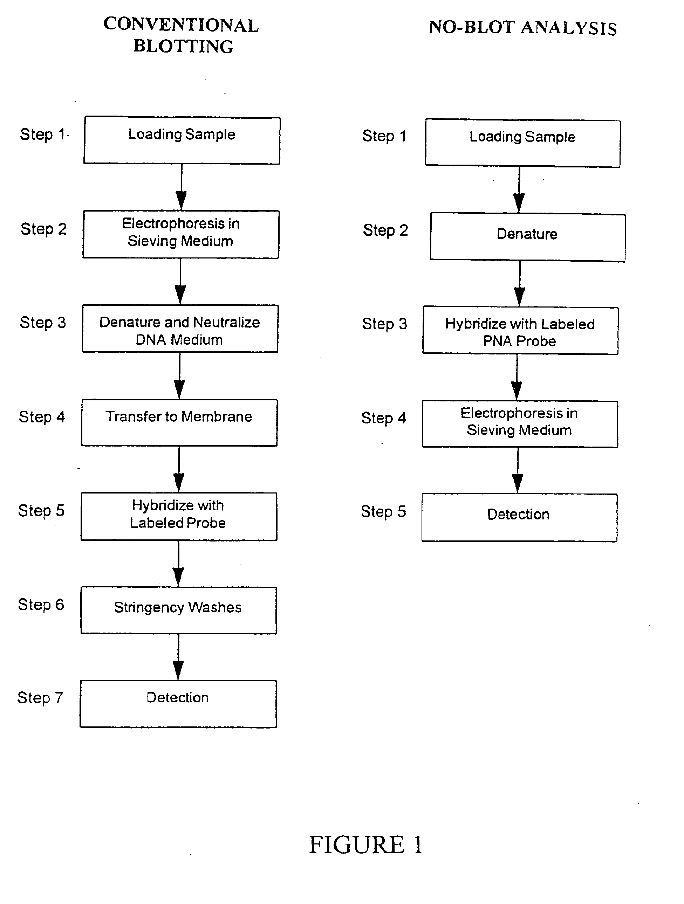

Thus, the claimed invention provides a method for the hybridization, separation and detection of molecules having a target sequence. Unlike traditional techniques, hybridization can occur simultaneously with, or prior to separation and detection. Conventional blotting with DNA or RNA probes involves a multi step procedure which is both time-consuming and labor intensive, requiring (1) loading a denatured sample onto a sieving medium; (2) separation by electrophoresis; (3) transfer to a membrane; (5) hybridization with a probe; (6) stringency washes; and, finally, (7) development of the label for detection. In contrast, the claimed methods require only hybridization with a probe, loading a sample onto a sieving medium or into the injection zone of a capillary, and separation by electrophoreseis prior to detection. Referring to figure 1, a flow chart diagram of the prior art process and a flow chart diagram of the process of the present invention are shown for direct comparison of their respective steps. Consequently the claimed methods are quicker, and less labor intensive than methods known in the art.

The following experiments reveal the utility of the PNA probe/nucleotide analysis compared to conventional DNA probe/DNA analysis.

### Example 1. Pre-Gel Hybridization

### A. Experimental Design:

Referring to Tables 1A and 1B, five different melting/annealing (hybridization) temperatures were tested using PNA and DNA probes labeled with biotin (samples A-E). The experimental controls included a DNA/PNA sample that was heated only at 94 °C; and a DNA/PNA sample that was heated only at 55°C. (Samples F1 and F2). At each temperature there were serial dilutions of the target DNA (i.e. 2 ng, 0.2 ng and 0.02 ng) (Table 1A). All samples had 1 picomole (pmol) of probe. The final salt concentration of the mixture was 1 mM Tris (7.5), 0.1 mM ethylenediaminetetraacetic acid (EDTA). (TE refers to the mixture of Tris and EDTA.) The total volume of each reaction was 10 µL

Hybridized samples were run on a 1X TBE (0.134 M Tris, 44.5 mM boric acid, 2.7 mM Na₂EDTA) 1% agarose gel. Capillary transfer to a non-charged nylon membrane was set up immediately after running gel. The transfer buffer was 20X SSC (3 M NaCl, 300 mM Sodium Citrate). The next day the membrane was dried and crosslinked with UV (ultraviolet light, in which the source delivers a total energy exposure of 33,000 µjoules/cm².) The membranes were placed in a heat sealable bag and the biotin label was detected using a chemiluminescent kit (# 7006) purchased from New England Biolabs. The manufacturer's directions were followed for this detection. The membrane was exposed to X-ray film and developed in order to attain the image.

**Table 1A.**

| Sample components | | | | | |
|---|---|---|---|---|---|
| Tube# | target DNA | biot.PNA | biot.DNA | TE | dH₂O |
| 1 | 2 ng (2 µL) | 1 pmol (1 µL) - | | 1 µL | 6 µL |
| 2 | 2 ng (2 µL) | - | 1 pmol (1 µL.) | 1 µL | 6 µL |
| 3 | 0.2 ng (2 µL) | 1 pmol (µL) - | | 1 µL | 6 µL |
| 4 | 0.2 ng (2 µL) | - | 1 pmol (1 µL) | 1 µL | 6 µL |
| 5 | 0. 02 ng (2 µL) | 1 pmol (1 µL) - | | 1 µL | 6 µL |
| 6 | 0.02 ng (2 µL) | - | 1 pmol (1µL) | 1 µL | 6 µL |

**Table 1B.**

| Experimental conditions | | |
|---|---|---|
| A) 94°C, | 10 min; | 45°C, 15 min |
| B) 94°C, | 10 min; | 55°C, 15 min |
| C) 94°C, | 10 min; | 75°C, 15 min |
| D) 94°C, | 10 min; | 45°C, 5 min |
| E) 94°C, | 10 min; | 75°C, 5 min |
| F1) 94°C, | 10 min | |
| F2) 55°C, | 10 min | |

The first temperature was designed to operate as a melting temperature and the second as an annealing temperature.

### B. Results:

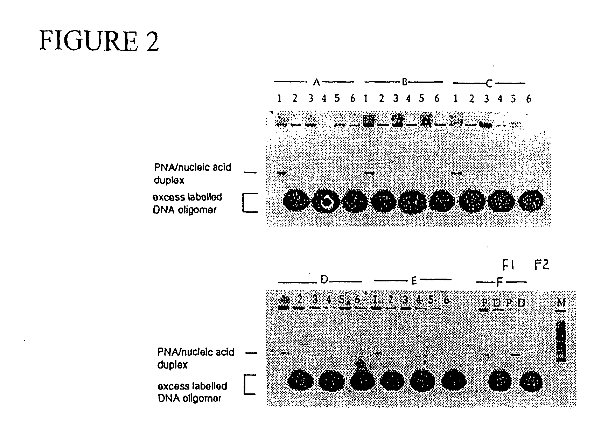

With reference to figure 2, a P indicates that a PNA oligomer probe was annealed (hybridized) and a D indicates that a DNA oligomer probe was annealed (hybridized) to the nucleic acid duplex. M indicates a marker lane. Lanes A1, B1, C1, D1 and El exhibit bands within the gel which represent the PNA probe/nucleic acid duplex which has migrated into the gel and is detected. Because each sample was exposed to differing conditions of temperature during the hybridization, the experiment demonstrates that the annealing phenomenum is fairly temperature independent. Under identical conditions, the analogous DNA probe/nucleic acid duplex is barely detectable; as indicated by lanes A2, B2, C2, D2,and E2. In each of these lanes, the labeled DNA probe is observed as a large circular blob at the bottom of each lane. This blob is visible because the excess DNA oligomer is charged and therefore migrates in the gel. The blob is at the bottom of the gel because it is small (15 nucleotides) and moves through the gel very rapidly. Conversely, the lanes with PNA probe exhibit background only near the loading well since the probes migrate into the gel primarily by passive diffusion. Detection of the PNA/nucleic acid duplex in lanes 3 and 5 of all samples required a longer exposure time for the film in order that the band be observed. Thus, there is a concentration dependence whereby the limits of detection are dependent on the starting amount of target DNA and the amount of probe available to bind to the target sequence. The controls demonstrate that the sample DNA/DNA melts even at 55°C which is also a suitable annealing temperature for DNA/PNA duplexes. Moreover, no annealing temperature need be attained since duplex is formed for control F1.

### Example 2. Pre-gel Hybridization Run on Different Gel Systems

### A. Experimental Design:

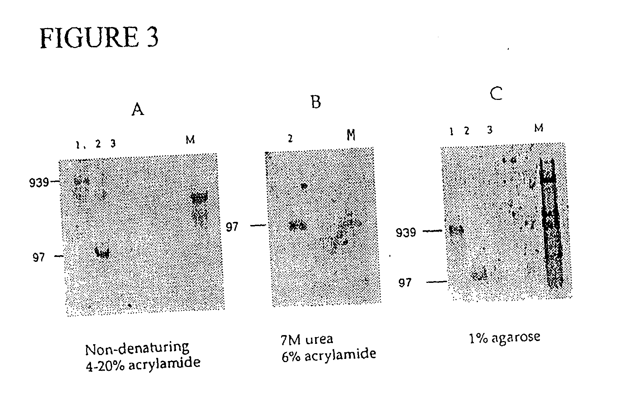

In this experiment each samples was prepared in triplicate. Two different restriction digests of a double stranded plasmid DNA were purchased for use in this experiment: 1) pBR322 digested with the restriction enzyme BstNI, 12 ng - to be split in thirds, and 2) pBR322 digested with the restriction enzyme MspI. Two separate starting concentrations were used for each sample of digested plasmid (i. e. 50 ng and 12 ng of starting plasmid). Each sample of plasmid digest was placed in an eppendorf tube and to that tube was added: 4 picomole (pmol) of the biotinylated PNA probe (5'-Bio-oo-ATGCAGGAGT CGCAT-3'), one-tenth volume of Tris-EDTA buffer, and deionized and filtered water (dH₂O) to bring the volume to 30 mL. These reactions were put at 70°C for 20 minutes. They were then split into thirds, the appropriate loading dye was added and they were separated on either a 4-20% non-denaturing acrylamide gel (figure 3A), 7M urea-6% acrylamide gel (figure 3B) or a 1% agarose gel (figure 3C). The components in each gel were then transferred by capillary action onto a non-charged nylon membrane. The membrane was dried and crosslinked with UV (ultraviolet light, in which the source delivers a total energy exposure of 33,000 µjoules/cm².) The membranes were placed in a heat-sealable bag and the biotin label was detected using a chemiluminescent kit (# 7006) purchased from New England Biolabs. The manufacturer's directions were followed for this detection. The membrane was exposed to X-ray film and developed in order to attain the image.

### B. Results:

With reference to figure 3, samples were only loaded on the gels where indicated by a numeral 1, 2, or 3. All numeral 1's were 4 ng of pBR322 digested with BstNI. All numeral 2's represent the higher concentration of 16 ng of pBR322 digested with MspI. Similarly, all numeral 3's represent the less concentrated 4 ng of pBR322 digested with MspI. The letter M indicates that a size marker was run in those lanes. As would be expected, for the different gel conditions, the mobility of the components varies substantially from gel to gel. With reference to figure 3 panel A, lane 1 contains a single stranded DNA fragment that is 939 base pairs (bp) long. The same band is apparent in lane 1 of figure 3 panel C. It is however, not seen in figure 3 panel B. This is because the fragment is too large to be successfully electrophoresed through 6% acrylamide. With regard to the PNA/fragment complex found in lane 2 of figure 3 panels A, B, and C there is one fragment of approximately 97 bp band that is visible. Again it is clear that different gels impart different mobilities on fragments of the same length. Lane 3 found in figure 3 panels A, B, and C, which is a more dilute solution of the fragment/PNA complex seen in lane 2, runs in the same part of each respective gel system.

This set of experiments demonstrates that the PNA/DNA complex is maintained/remains intact in a 7M urea-6% acrylamide gel and in the formamide used in the loading dye. If this were not the case then no distinct band would be visible because the detection depends on the biotin label that the PNA carries. Note that the only visible band on the 6% acrylamide gel is the pBR322 MspI band which is approximately 97 bp. This band is significantly shorter than the BstNI target (939 bp). The band is visible because the 6% acrylamide gel separates fragments shorter than 500 bp. Larger fragments do not enter the gel, nor are they substantially transferred to a membrane.

### Example 3. Restriction Enzyme Digest/Pre-gel Hybridization

### A. Experimental Design:

Six sets of reactions were set up in duplicate. 4 ng of linearized double stranded pBR322 was used for each reaction. To the pBR322 was added 2pmol of a biotinylated PNA, 1/10th vol of TE buffer and dH₂O to bring the reaction volume to 10 µL. Sets of samples were incubated at the indicated temperature for 10 minutes, and then slowly (over 30 minutes) cooled to room temperature. (Sample 2B was heated initially at 94°C, sample 3B and D heated at 85°C, sample 4B and D -75°C, sample 5B and D -65°C, sample 6B and D -55°C, and sample 7B and D were left at room temperature.) After all samples had slowly cooled to room temperature, dH₂O, 1/10th volume of medium salt (NEB 2- 10 mM Tris-HCl, 10 mM MgCl₂, 50 mM NaCl, 1 mM dithiotheritol) and 1 ml of Pst I restriction enzyme were added to each sample. These were incubated at 37°C for 20 minutes and then left overnight at ambient temperature. The next day, each tube was given an additional 0.5 µL of restriction enzyme and incubated at 37°C for 15 minutes. With reference to figure 3, one set of samples (B) had loading dye added and were ready to load on the agarose gel. The other set (D) was incubated at 94°C for 10 minutes. Then loading dye was added to the D set and the samples were loaded. All samples were electrophoresed through a 1 % agarose gel. The components in each gel were then transferred by capillary action onto a non-charged nylon membrane. The next day the membrane was dried and crosslinked with UV (ultraviolet light, in which the source delivers a total energy exposure of 33,000 µjoules/cm².) The membranes were placed in a heat-sealable bag and the biotin label was detected using a chemiluminescent kit (# 7006) purchased from New England Biolabs. The manufacturer's directions were followed for this detection. The membrane was exposed to X-ray film and developed in order to attain the image.

### B. Results:

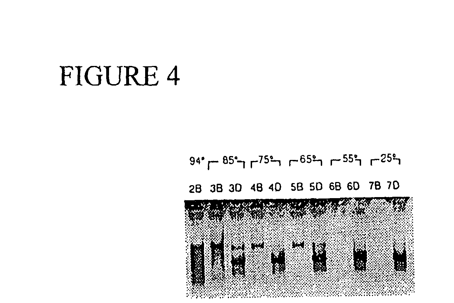

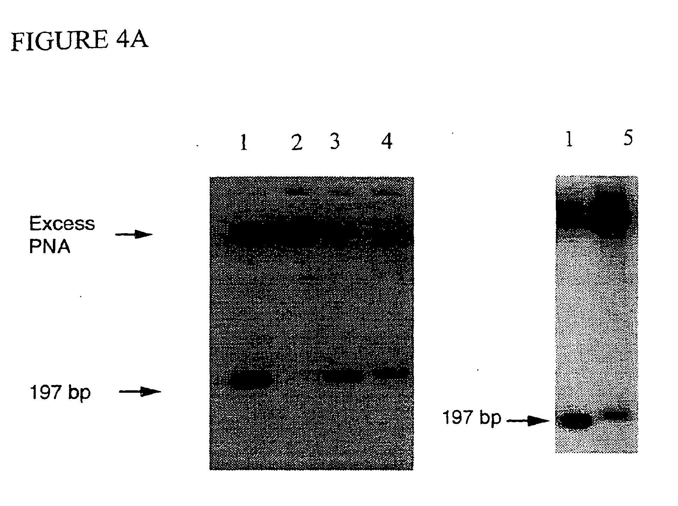

With reference to figure 4, the only lanes in which there is visible digested DNA (i. e. a shorter fragment is visible) are observed in the D lanes for each sample. This demonstrates that in order for the DNA to be digested, it must first be reannealed so that it is double stranded. When the reannealling occurs the PNA probe is displaced from the complex. The B lanes contain digested DNA fragments which are not detected because the PNA probe was displaced from the complex and so the DNA is no longer labeled. The D lanes are visible because after digestion, the PNA probe had a chance to reanneal because the 10' treatment at 94 °C melted the double standard DNA so that the PNA could once again bind. Figure 4 demonstrates several characteristics of the PNA probes and double standard DNA. First, it is preferable to melt the DNA at high temperatures, as the reannealing takes substantially longer as the melting temperature increases. Second, only DNA given the chance to have a PNA reanneal to the single stranded DNA, is seen digested. For 65, 75, 85 and 95 °C, there is some single stranded DNA remaining with a PNA bound to it. This is not true for either the 55 °C or room temperature sample. Third, since the samples were not reheated after digestion in the presence of PNAs we concluded that there is cut DNA present but it is not detected. Our overall conclusion is that the DNA that we visualize on these gels is single-stranded, and that when the DNA renatures, which is a slow process, it displaces the PNA.

### Example 4. Pre-gel Hybridization Assay for Cystic Fibrosis Gene W1282

### A. Experimental design:

The W1282X mutation in cystic fibrosis carriers was used to test the utility of the method for the detection of a single base mutation in a clinically relevant assay. In Referring to figure 4A, four hybridization assays were run in parallel using a DNA sample provided by an unaffected individual containing two wild type alleles W1282, lanes 1 and 2, and a DNA sample provided by a carrier containing a wild type allele W1282 and the mutant allele W1282X, lanes 3 and 4, using a fluorescein labeled PNA probe for the wild type, Flu-oo-CTTTCCTCCA CTGTT-NH₂, lines 1 and 3, and a fluorescein labeled PNA probe for the mutant, Flu-oo-CTTTCCTTCA CTGTT-NH₂, lines 2 and 4. Each target DNA sample (0.5-1.0 µL) from a PCR reaction was mixed with the fluoresceinated PNA probe (0.5-1.0 pmol) and 1 µL of 250 mM Tris (pH 8.0) and the total volume was brought to 10 µL with dH₂O. Each mixture was heated at 95°C for ten minutes then cooled to ambient temperature for ten minutes. Loading dye was added and each mixture was loaded on a 1.5% TBE agarose gel and electrophoresed for twenty five minutes. The gel was prepared for a Southern transfer to a nylon membrane. The membrane was dried and crosslinked at 254 nm before detection. Detection of chemiluminescence of the fluoresceinated PNA probes and PNA/DNA hybrids was carried out by placing the crosslinked membrane in a heat-sealable bag with an anti-fluorescein antibody directly conjugated with alkaline phosphatase DAKO (#K046, Copenhagen, Denmark). The membrane was rinsed in five volume TMS (10 mM Tris, pH 9.5, 10 mM NaCl, 1 mM MgCl₂) for two minutes and Lumigen solution (Prototpe #7006, New England Biolabs). The membrane was exposed to Fuji RX film.

### B. Results:

Referring to figure 4A, a very efficient discrimination was observed even though stringent conditions could not be applied through controlled temperature. The intensities of the bands from the samples provided by the carrier, lanes 3 and 4, were approximately half the intensity of the band resulting form the wild type PNA/DNA hybrid, lane 1. The mutant PNA probes did not hybridize with the wild type DNA sample, lane 2, demonstrating the high specificity of the method as applied to single base pair discrimination of the sample DNA. In all of the lanes, excess labeled PNA probe was observed with varied intensity, the most intense being observed in lane 2, where the mutant PNA probe did not form detectable amounts of hybrid with the wild type DNA sample. The efficiency in single base pair discrimination observed are due to two factors: 1) the labeled PNA probes are intrinsically highly specific : and 2) the relatively short length of the PNA probes, and most importantly only a small excess of probe is used for the assay. This is in contrast to the traditional Southern blotting in which a high excess of (DNA) probe is normally used. When an excess of the mutant PNA probe was used with the wild type DNA sample, a false positive was observed, lane 6.

### Example 5. Rapid Acquisition of Southern Blot Data by Capillary Electrophoresis

### A. Capillary Electrophoresis (CE) Instrumentation:

A P/ACE 2050 capillary electrophoresis instrument with a Laser Module 488 argon ion laser (Beckman Instruments, Fullerton, CA, USA) was used with a 520 nanometer (nm) bandpass filter for laser induced fluorescence (LIF) detection. Ultraviolet (UV) absorbance detection was performed at 260 nm. Separations were performed in the negative polarity mode with the cathode on the injection side. The capillary was a 75 µm inside diameter (i.d.) untreated capillary (35 cm in length and 28 cm to the detector) (P/N 2000017, Polymicro Technologies, Phoenix, AZ, USA) with 35 centimeter (cm) in length (28 cm to detector).

### B. Materials:

A commercially available plasmid (M13mp18 DNA) and the BstN1 (P/N #303-1S) and Msp1 (P/N #303-2L) restriction digests enzyme plasmid pBR322 were obtained from New England Biolabs, Beverly, MA, USA. These served as nucleic acid samples having known target sequences of interest. Tris-Borate-Ethylenediaminetetraacetic acid (TBE) buffer powder was obtained from Sigma (St. Louis, MO, USA) and 1x TBE (pH 8.3) was prepared. 1% Poly(ethylene oxide) (PEO) (MW 4 million dalton) polymer buffer solution was prepared with 1xTBE buffer.

Fluorescein or biotin labeled PNA probes having both completely complementary sequences and base pair mismatches to the targets were synthetized on an Expedite Nucleic Acid Synthesis System from PerSeptive Biosystems (Framingham, MA, USA). Spacers units of 8-amino-3,6-dioxaoctanoic acid (-o-) were added to the resin-bound PNA before reacting activated esters of biotin (Bio) or fluorescein (Flu) such as dimethoxytritylbiotin ester of 1(4'-nitrophenyl)pyrazolin-5-one (DMTr-bio-HPP) or 5,6-carboxyiluorescein-N-hydroxysuccinimide. The labeled PNA was then cleaved from the resin and protecting groups removed using TFMSA/TFA/m-cresol/thioanisole (2:6:1:1) mixture for two hours at room temperature. The labeled PNAwas precipitated from the filtrate by addition of anhydrous ether. The crude PNA precipitate was purified by HPLC on a Deltapack C18 column (Waters) and by Sephadex G-25 to remove fluorescent impurities. The sequences of the various labeled PNA probes used in the following experiments are listed in Table 2

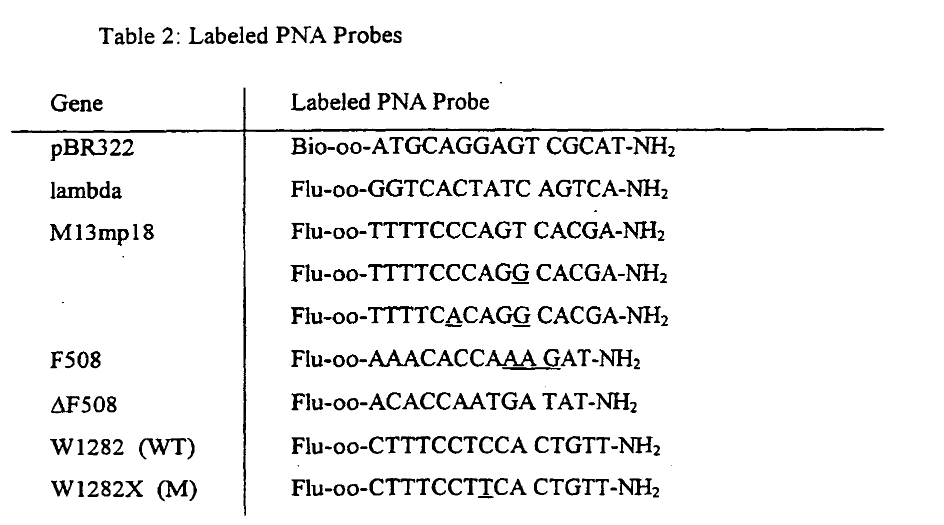

### C. Experimental Design (Probe Hybridization)

10 microliter (µl) of a solution containing the DNA target of interest (10⁻⁷-10⁻⁸ M nucleic acid sample) and 10 µl of a sample containing the labeled PNA probe (10⁻⁶-10⁻⁷M) was added into a microvial. The mixture was heated at 90°C for 10 minutes to melt any double-stranded stretches of DNA (secondary structure) and was then cooled to ambient temperature. The sample was then loaded onto the capillary electrophoresis apparatus and the separation was run. Detection capability is integrated into the instrument described above.

### D. Results:

### Experiment 1. Temperature Control for PNA/DNA Hybridization

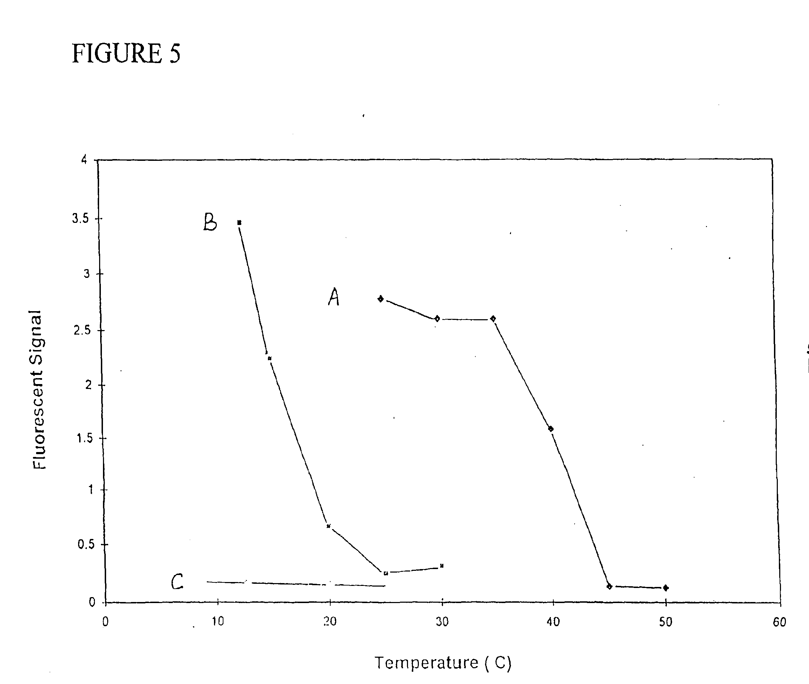

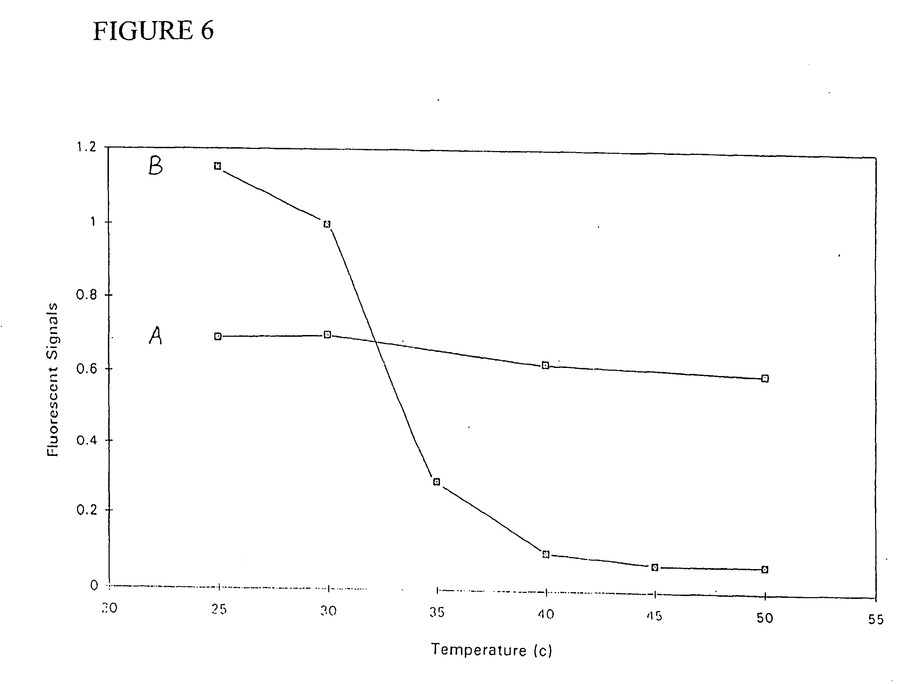

Temperature control experiments for hybridization of fluorescein labeled PNA probes with complementary Flu-oo-TTTTCCCAGT CACGA-NH₂, one-base mismatch Flu-oo-TTTTCCCAGG CACGA-NH₂ and two-base mismatch Flu-oo-TTTTCACAGG CACGA-NH₂ sequences to the M13mp18 plasmid were performed. Samples were prepared as described above and analyzed on the instrument described above. For this experiment, the capillary was placed in a cartridge which was thermostatted thereby facilitating temperature control during the separation. A series of CE separations were performed under a potential of 20 kilo volt (KV) at temperatures ranging from 10°C to 50°C. The analysis was carried out in the presence (figure 5) and the absence (figure 6) of 30% formamide in 1x TBE buffer (pH 8.3). LIF detection was performed with 488 nm for excitation and 520 nm for emission. Generally, it was observed that the melting points of the hybrids decreased with increasing number of base pair mismatches, this being an expected result.

Data obtained in the absence of formamide is presented in figure 6. With reference to figure 6, Curve B represents data obtained for the two-base mismatch and Curve A represents data obtained for the complementary PNA probe. Because Curve A is relatively flat the data indicates that the duplex formed from the complementary probe is stable at all temperatures recorded. The sigmoidal shape of Curve B is typical for a thermal melting profile and indicates that the probe having 2 base pair mismatches substantially anneals to the target at temperatures below 30°C and the melting point of the PNA/DNA duplex is about 33°C.

Data obtained in the presence of 30% formamide in the running buffer (a denaturing reagent) is presented in figure 5. Represented are the temperature control curves for the samples containing a complementary PNA probe (Curve A), one-base mismatch PNA probe (Curve B) and two-base mismatch PNA probe (curve C). The low signal level and the lack of slope to Curve C indicates that the probe having a two base mismatch does not anneal to the target within the range of temperatures used in the experiment. The slope of Curve B indicates that the probe having a single base mismatch anneals to the target but the complex completely dissociates above 25°C. Curve A indicates that the melting temperature (Tm) of the complex formed under these conditions with the complimentary PNA probe is between 35 to 38°C since the fluorescence signal started to drop sharply at 35°C. The PNA probe assay can be performed at a higher temperature and at denaturing condition to increase the specificity of the assay.

### Experiment 2. Capillary Gel Electrophoresis for DNA Size Separations

An uncrosslinked polymer (PEO) was utilized as a sieving media in capillary gel electrophoresis (CGE) for DNA separations. There is a phenomenon in capillary electrophoresis wherein ionization of the silanols on an uncoated capillary surface creates an electric "double layer" of buffer cations across the surface. When voltage is applied across the capillary, migration of the double layer of cations causes a flow of bulk fluid in the capillary toward the cathode. This flow is the result of electroendoosmosis and is called electroosmotic flow (EOF). It is desirable to minimize EOF in capillary gel electrophoresis for DNA separation. The advantage of the polymer gel-filled untreated capillary is that EOF was minimized, and the low viscosity polymer solution can be pushed out of the capillary under pressure and then replaced easily.

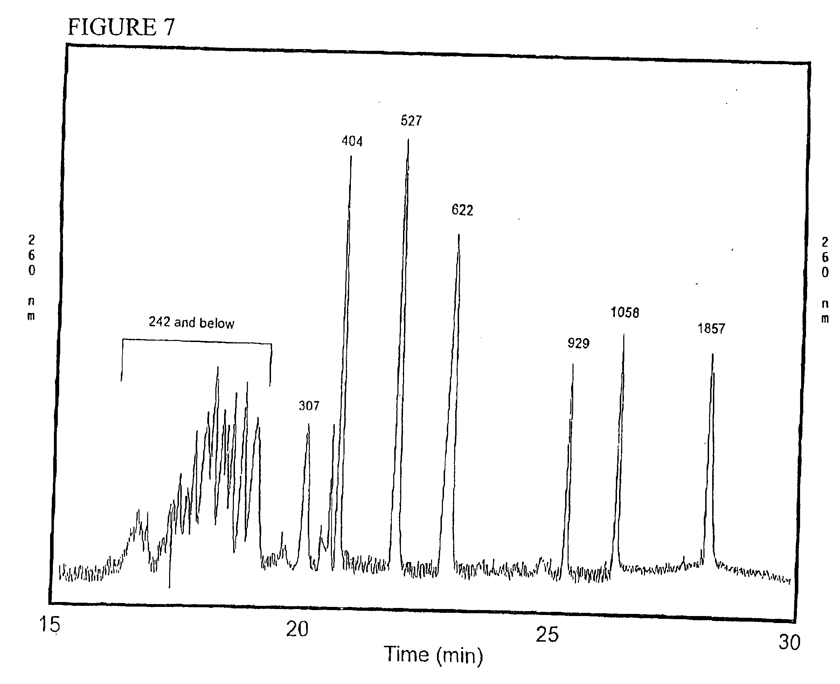

Various molecular weights of PEO polymer were tested. Electroosmotic flow was very low with a 0.5% PEO (MW 8 million dalton) filled uncoated capillary. Consequently, using this model system, the BstN I and Msp I digests were separated by the PEO gel-filled capillary (figure 7). A potential of 10 KV was used for all separations. Detection of the sample components was performed by UV detection at 260 nm.

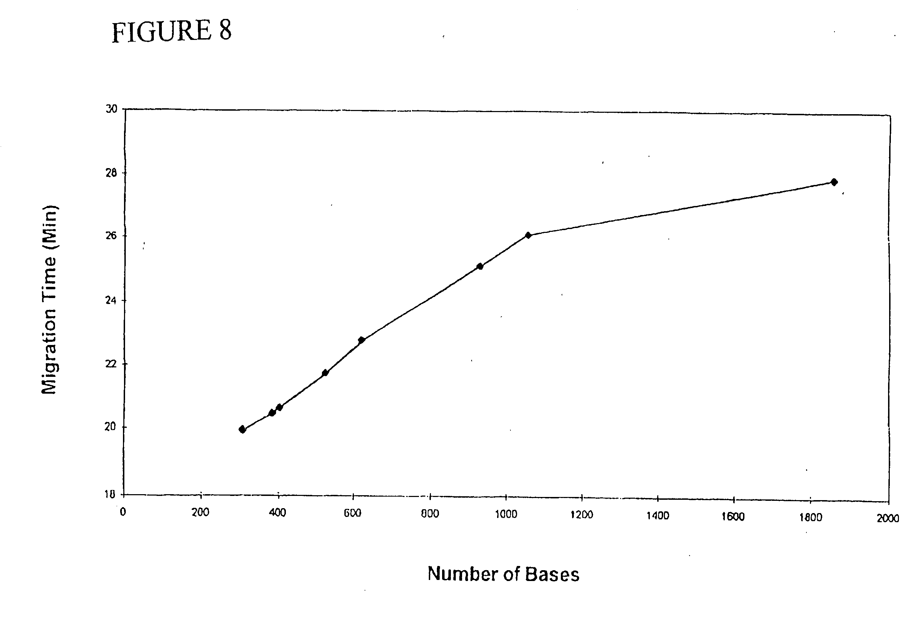

The peaks were compared with the bands obtained from an agarose slab gel electrophoretic separation of the same sample. Known peaks were assigned based on analysis of the two separations and the known molecular weights as supplied by the manufacturer. Figure 8 represents the calibration curve of the BstN1 and Msp1digest samples. Migration time was plotted against the length of the nucleic acid fragments. The data was quite linear from 300 bases to 1200 bases. The coefficient of variation (CV), standard deviation divided by the average, were less than 0.65% for six replicate runs of Msp1 fragment separations. The data indicated that the capillary would perform an adequate size separation of the fragments.

### Experiment 3. Probe Hybridization and Size Determination by CE Analysis

A fluorescein labeled PNA 7.5 10⁻⁷M probe having a sequence complementary to a target sequence of the pBR322 DNA sample was prepared as described above. Both the Msp I (1000 µg/mL) and the BstNI digest of pBR322 DNA (1000 µg/mL) were used as targets. The target sequence and the sequence of the PNA probe are shown below. 
PBR322 DNA: 5'------------------ATGCGACTCC TGCAT---------3'
PNA Probe: H2N-TACGCTGAGG ACGTA-oo-Flu
 Homogeneous solution hybridization was done by mixing the fluorescein-labeled PNA (in excess) with the pBR322 DNA digests. The mixture was then heated to 90°C for 5 min to denature the double-stranded DNA. The samples were then allowed to cool to ambient temperature. The mixture was then separated by capillary gel electrophoresis with LIF detection using the capillary and apparatus described above.

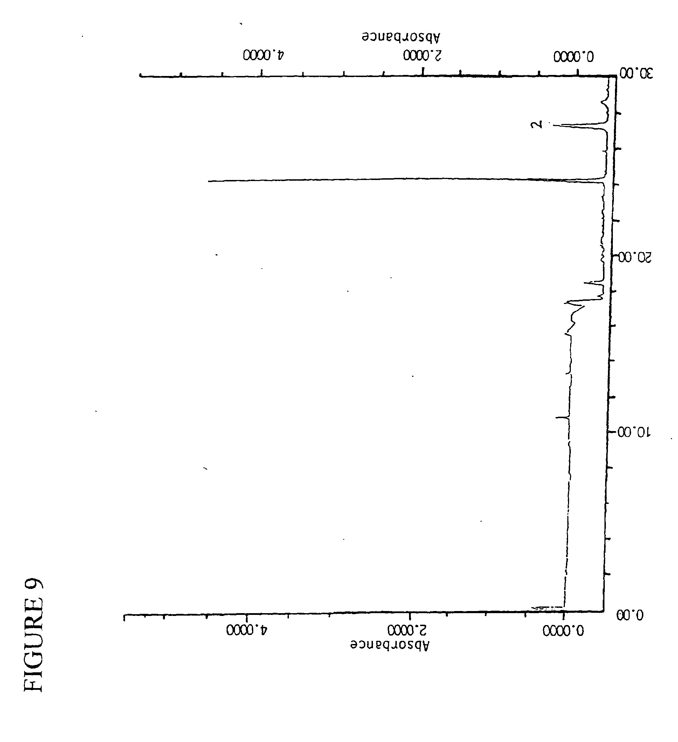

With reference to figure 9, the PNA/nucleic acid duplex formed from the labeled PNA probe and the nucleic acid fragment (929 bases) of the BstN I digest bearing the target sequence can be detected (Peak 1). Because the fluorescein label of the labeled PNA probe carries a negative charge, the labeled PNA probe migrates into the gel and is also detected (Peak 2).

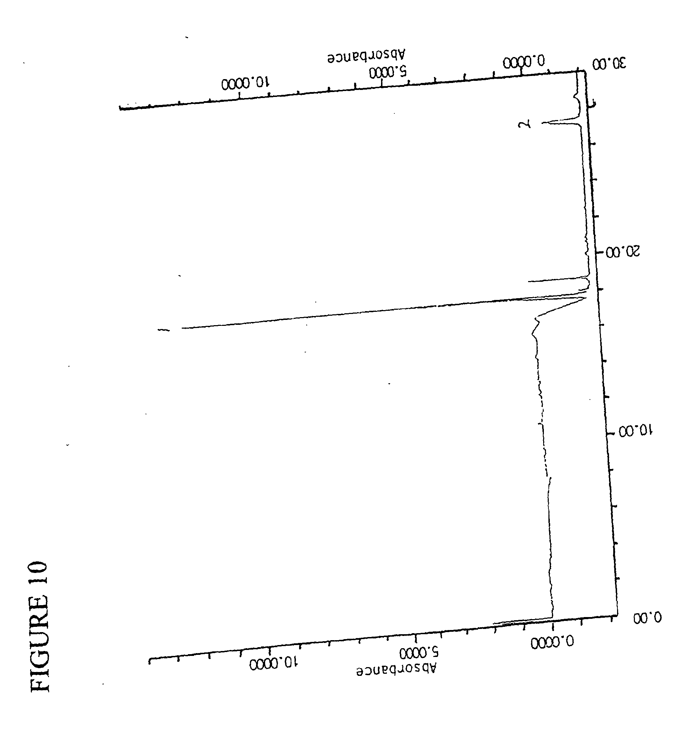

With reference to figure 10, the PNA/nucleic acid duplex formed from the labeled PNA probe and the nucleic acid fragment (97 bases) of the Msp I digest bearing the target sequence can be detected (Peak 1). Because the fluorescein label of the labeled PNA probe carries a negative charge, the labeled PNA probe migrates into the gel and is detected (Peak 2).

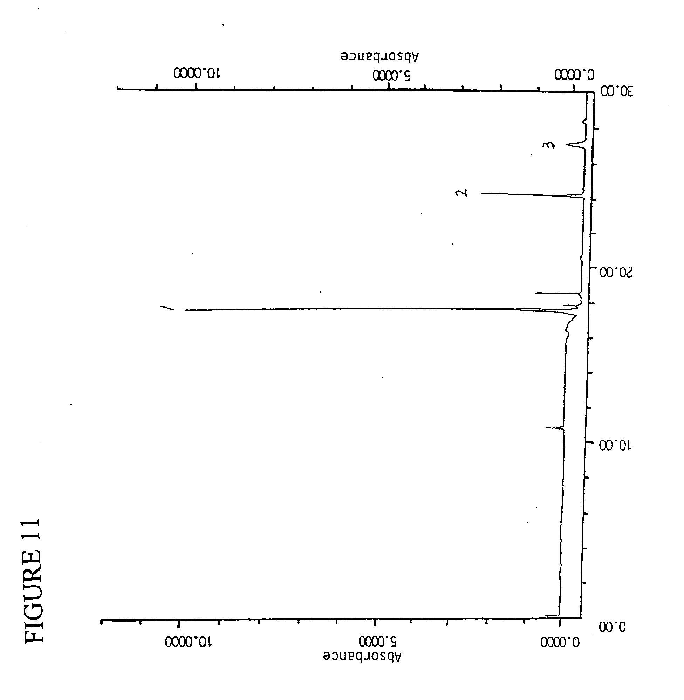

With reference to figure 11, a comparative separation of samples referred to in figures 5 and 7 demonstrates that a comparative size separation of the fragments bearing the target sequences can be made. Thus, peak 1 is a duplex formed from the Msp1 fragment (97 bases) bearing the target sequence and the labeled PNA probe. Peak 2 is a duplex formed from the BstN I fragment (929 bases) bearing the target sequence and the labeled PNA probe. Peak 3 is again the excess fluorescein labeled PNA probe. The fragments containing the complementary sequence were separated and specifically detected by CGE-LIF within 30 min. Consequently, in one 30 min run, both size and sequence information is obtained for the samples. High throughput can be achieved by using a microchip format where the analyses are performed in channels formed in a solid substrate. Such devices can be made with multiple channels enabling many separate analyses to be performed in parallel. See Stu Borman, Developers of Novel DNA Sequences Claim Major Performance Advances, *Chemical & Engineering News,* July 24,1995, pp. 37-39.

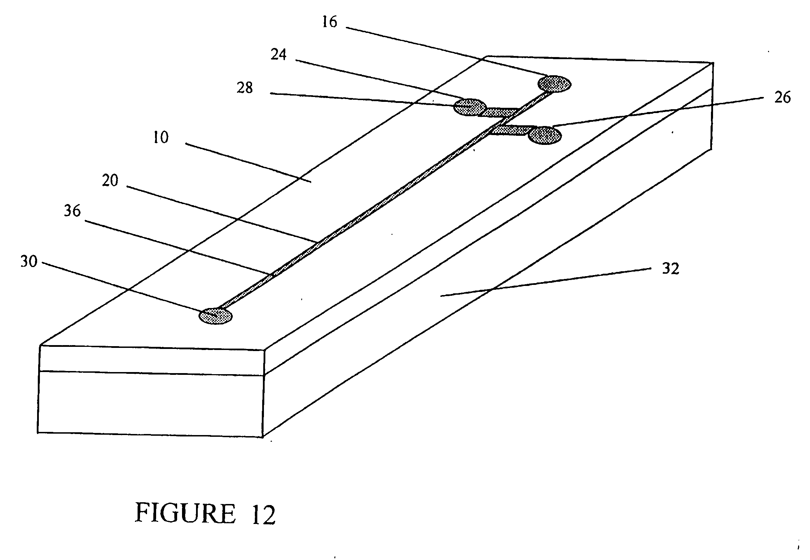

In another aspect, the claimed invention provides an apparatus for detecting the presence, absence or concentration of an analyte in a sample. The apparatus may, in various embodiments, comprise, for example, a sample injection zone, a PNA probe disposed to mix with a sample introduced to said injection zone, and a separation zone. In some embodiments, the apparatus may comprise a sample incubation zone. Referring to figure 12, in certain embodiments, the apparatus 10 comprises a sample injection zone 24. One or more PNA probes 28 are disposed within the sample injection zone 24, such that, upon introduction of the sample into zone 24, the probes 28 come into contact with the sample. If the target is present, then the probe will hybridize with said target. The apparatus further comprises a capillary or channel 20 having disposed therein a separation medium 36, the channel being in communication with the sample injection zone 24, a buffer reservoir 16, an injection/waste reservoir 26, and a waste reservoir 30. In certain embodiments, the junction of the sample injection zone 24 with the capillary 20 is upstream from the junction of the injection/waste reservoir 26 with capillary 20. This offset design allows to define a larger injection volume.

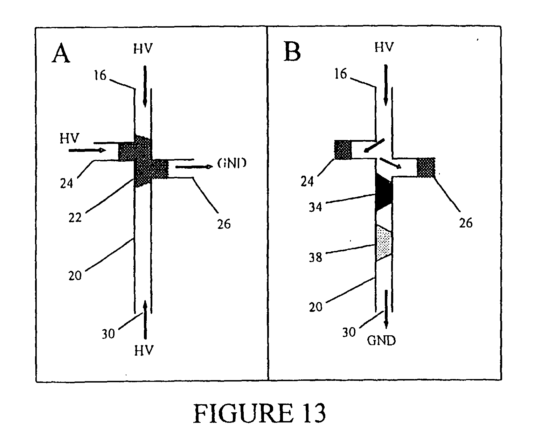

In operation and referring to figure 13 panel A, a sample is added to the injection zone 24, and a target, if present, will hybridize with probe 28. A high voltage (HV) is applied to the zones 16, 24 and 30 relative to the ground (GND) applied to zone 26 and is controlled to pinch off the flowing sample stream, preventing diffusion of sample 22 into the separation channel 20 providing an injection volume which is independent of sampling time. The electrical potential between zones 16, 24, 30 and 26 is then shut off, and a potential is imposed between zone 16 and zone 30 to separate the components of the sample by size and electrophoretic mobility, for example, components 34 and 38 shown in figure 13 panel B.

In preferred embodiments, the apparatus may be disposed in contact with a temperature controlling means 32 (figure 12). The temperature controlling means 32 can be activated to melt or denature double stranded samples in the injection zone, as well as to render the separation medium 36 denaturing. In some embodiments, one may add sample to sample zone 24, adjust the temperature controlling means to denature the sample, and adjust the temperature controlling means 32 again to allow hybridization with the probe 28 to occur. In some instances it may be desirable to the adjust the temperature controlling means 32 again to render the separation medium 36 denaturing.

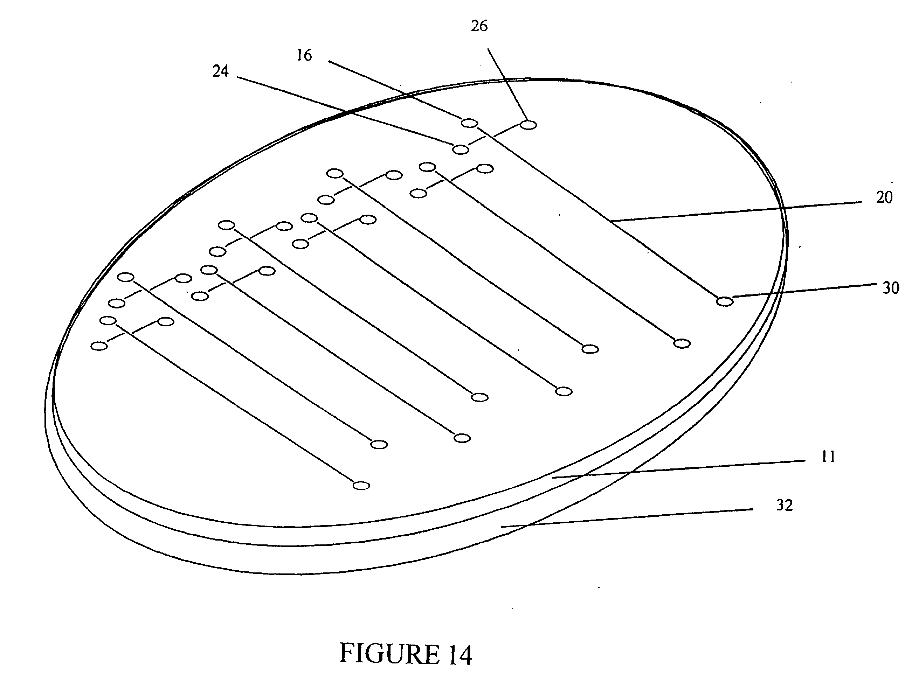

In other embodiments, the apparatus is a microchip having multiple parallel channels formed in solid substrates. Such devices allow many separate analyses to be performed in parallel, achieving thereby a high throughput. In brief overview and referring to figure 14, the apparatus 11 comprises multiple capillary channels 20 each having a sample injection zone 24, a buffer reservoir 16, an injection/waste reservoir 26, and a waste reservoir 30. In preferred embodiments, the apparatus may be disposed in contact with a temperature controlling means 32. Any known detection means may be utilized in the apparatus of the invention, and can easily be selected by one skilled in the art. When utilized in accordance with the methods and compositions of the instant invention, the apparatus permits qualitative and quantitative detection of an analyte in a sample. Such microchip separation devices are made using photolithography and chemical etchants to produce channel structures in a fused silica wafer. Access holes are laser drilled at channel terminals through the etched wafer. A second fused silica wafer is bonded to the etched wafer to produce enclosed channels. After bonding, the wafer is cut into individual separation chips.

In more detail, to perform the microfabrication, a film of chromium (400 A) is sputtered onto the fused silica substrate (75 mm diameter x 0.4 mm; Hoya, Tokyo, Japan). Photoresist (Shipley 1811, Newton, MA) is spin-coated onto the wafer 11 and baked at 90 °C for 25 min. The resist is patterned by exposing it to UV (365 nm) radiation through a contact-aligned photomask (Advanced Reproductions, Wilmington, MA) and developing it in Microposit developer (Shipley). The chrome is removed using K₃Fe(CN)₆/NaOH (Chrome Etch, Shipley). The resulting mask pattern is etched into the fused silica by immersing the wafer 11 in NH₄F/HF (1:1) etchant at 50 °C. The depth of etching is controlled by monitoring etching time and measured with a profilometer (Mitutoyo). The microchip utilized in this study is etched to a depth of 28 µm, yielding a channel width 20 of 66 µm at the top of the channel because of the isotropic etching conditions. The cross-sectional area of the channel 20 is equivalent to that of a cylindrical capillary with an i.d. of 44 µm. Photoresist is removed with acetone, and the remaining chrome is dissolved using K₃Fe(CN)₆/NaOH.

Access to the channel terminals is provided by laser-drilled holes through the etched wafer 11. A second fused silica wafer is bonded to the etched wafer to enclose the channels. Both wafers are immersed in 50 °C NH₄OH/H₂O₂ and rinsed in H₂O. They were then placed in contact and thermally bonded. Initial bonding took place at 200 °C (2 h), followed by final bond formation at 1000 °C (overnight). Individual reservoirs 16, 24, 26 and 30 cut from glass tubing were attached with silicone adhesive (Dow Corning).

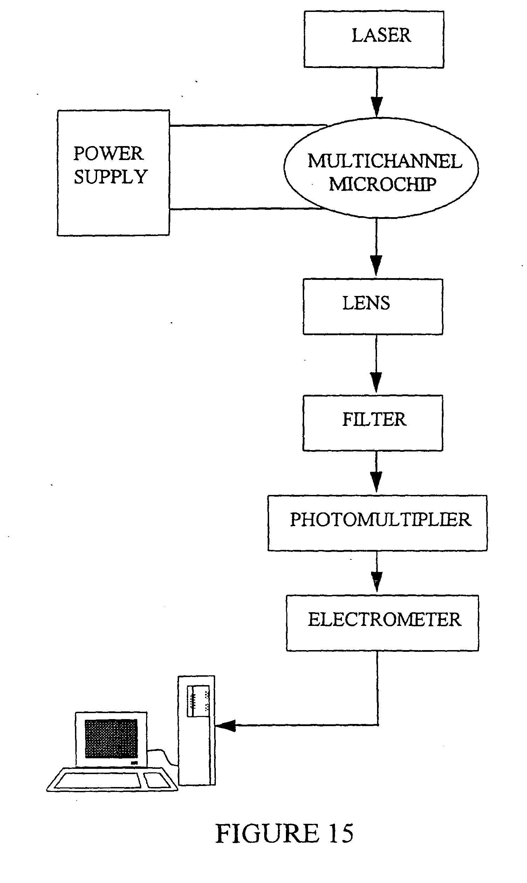

Referring to figure 15, such a fused silica microchip is coupled with a power supply and fluorescence optics for the chip-based assay. High voltage is provided by a Spellman CZE 1000R power supply (Plainview, NY) or other suitable supply through a switching circuit and resistor network. Laser-induced fluorescence detection is performed using an Omnichrome (Chino, CA) argon ion laser operating with -3 mW of excitation at 488 nm, or other suitable device, focused into the channel at a 53° angle of incidence with a 10 cm focal length lens. A 20x microscope objective (Edmund Scientific, Barrington, NJ) collects fluorescence emission. The collected light is spatially filtered by a 2 mm i. d. aperture in the image plane and optically filtered by two 520 nm bandpass filters (520DF30) Omega Optical, Brattleboro, VT). A photomultiplier tube (such as Hamamatsu R928, Bridgewater, NJ) connected to an electrometer (such as a Keithley 614, Cleveland, OH) detects the fluorescence signal. The signal is digitized with a PC-controlled 20 bit data acquisition system (such as a Data Translation 2804, Marlborough, MA) and anlyzed using appropriate software (such as Caesar software from ADI, Alameda, CA).

Any conventional method of detection may be used in the apparatus of the invention, including those used in more conventional electrophoresis methods. A detection method is chosen which allows for detection of any suitably detectable physical property of a species. These detection systems include, but are not limited to, absorbance of ultraviolet or visible radiation, fluorescence, refractive index, Raman, mass spectrometry, chemiluminescence, electrochemical, and conductivity.

In yet another aspect, the claimed invention provides kits for detecting the presence, absence or concentration of a target sequence using electroseparation analysis. Preferred embodiments of kits are configured to detect preselected target sequences in having various samples. Specifically, the kits of the invention may include containers housing having a preselected denaturing sieving medium and a PNA probe of the invention. The kits of the invention may, in some embodiments, contain more than one labeled probe which can anneal to one or more different target sequences which are unique to a genetic characteristic of interest in a nucleic acid sample. The labels on the probes may be the same or different, and may be independently detectable. In certain embodiments, the kits of the present invention further provide an electroseparation apparatus comprising a channel or a plurality of channels. It is contemplated that the kits of the invention optionally provide a disposable apparatus, comprising a capillary or plurality of capillaries as defined herein. The benefits of such a micro channel apparatus is seen from the following experiments.

### Example 6 PNA/DNA Assay on a Microchip

### A. Experimental Design

A fluorescein labeled PNA probe was prepared as described above. The labeled PNA probe is a 15mer with a complementary sequence to PBR322 DNA (MW 4774 determined by mass spectrometry). The mixture of the two restriction enzyme digests, BstNI and MspI, of pBR322 DNA were used as targets.

PBR322 DNA: 5'---------ATGCGACTCCTGCAT-------------3'
PNA Probe: H2N-TACGCTGAGGACGTA-oo-Flu

Homogeneous solution hybridization was done by mixing the fluorescein-labeled PNA (in excess) with the pBR322 DNA digests, heated it at 90 °C for 5 min. to denature the double-stranded DNA and cooled it to room temperature. The experimental procedure is describe above in Example 4, subsection 3. The mixture was separated by microchip gel electrophoresis with LIF detection applying 704 v/cm voltage. The channels' length was 22 mm and the channels' cross section is about 100 µm x 50 µm.

### B. Results

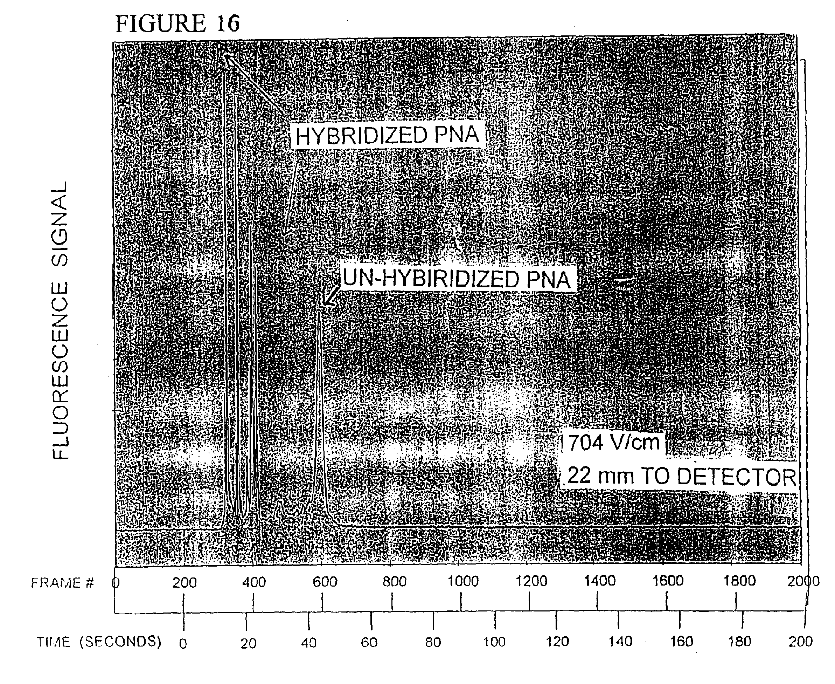

With reference to figure 16, the size of the hybrid of the fluorescein labeled PNA and the target DNA fragment can be detected. Since the PNA is labeled with fluorescein, the PNA Probe is carrying a negative charge which comes from the negatively charged fluorescein. As a result, the fluorescein labeled PNA will move in the capillary but not as fast as DNA or PNA/DNA hybrids, towards the anode in an electric field when it is injected from the cathode. Figure 16 shows an electropherogram of a PNA probe hybridization determination of pBR322 DNA Msp1 and BstN1 digests. The formation of PNA/Msp1 fragment (97 bases) hybrid duplex and PNA/BstN1 fragment (929 bases) hybrid duplex are detected within 30 seconds. The PNA in excess is detected after 40 seconds. The fragments containing the complementary sequence were separated and specifically detected by LIF within 30 seconds.

### Example 7 Cystic Fibrosis F508 Genes

### A. Experimental Design

Cyclic Fibrosis (CF) is a common lethal ressessive disorder. A major mutation causing the disease (ΔF508) has been found in 70% of the CF chromosomes. The experimental procedure is describe above in Example 5. This ΔF508 mutation is a three base deletion. The following PNA probes for the wild type (F508) and the mutant gene (ΔF508) were prepared:

F508 Flu-oo-AAACACCAAA GAT-NH2

ΔF508 Flu-oo-ACACCAATGA TAT-NH₂

### B. Results

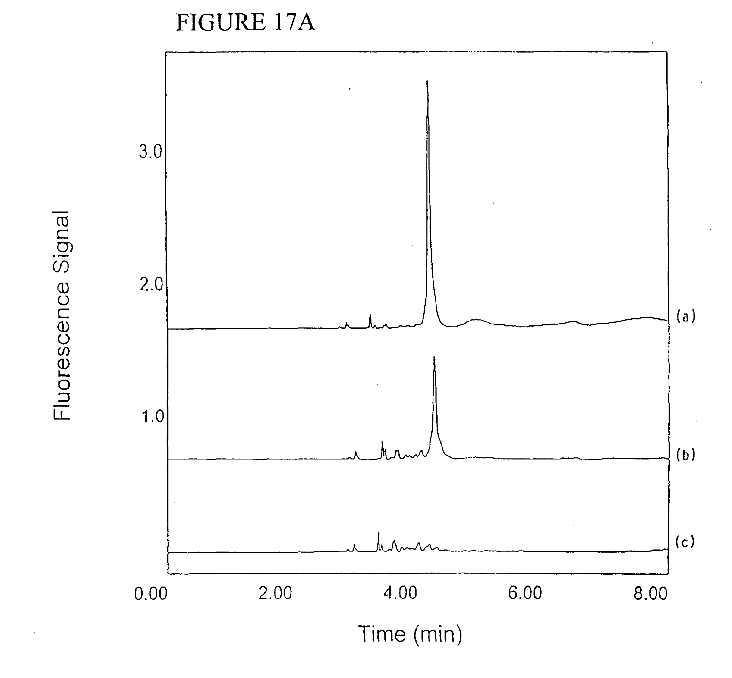

A hybridization assay with a labeled PNA probe for the wild type Cystic Fibrosis F508 gene was conducted by capillary electrophoresis with three DNA samples provided by: (a) an unaffected individual; (b) an unaffected carrier; and (c) an affected patient. The results are presented in figure 17A. All things being equal, and the three assays being run simultaneously in parallel, figure 17A shows that the maximum signal is collected from the sample provided by the unaffected individual indicating that both genes annealed to the wild type labeled PNA probe; a less intense signal is collected from the sample provided by the healthy carrier indicating that only one of the two genes, the wild type one, annealed with the wild type labeled PNA probe, while the mutant ΔF508 gene did not, and no significant signals were detected from the sample provided by the affected patient indicating that no wild type genes were available from the sample.

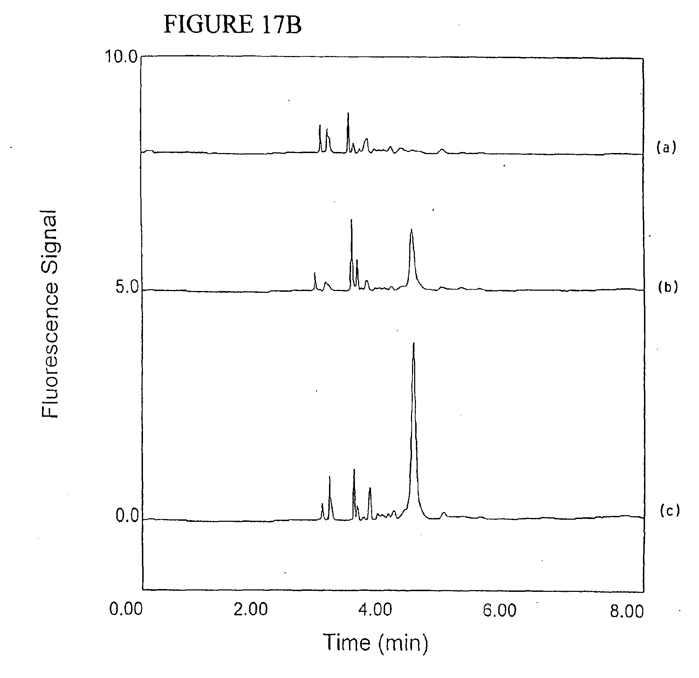

In a second hybridization assay, a labeled PNA probe for the mutant ΔF508 was used to allow direct detection and identification of the mutant gene ΔF508. The assay was conducted by capillary electrophoresis with three DNA samples provided by: (a) an unaffected individual; (b) an unaffected carrier of the ΔF508 mutation; and (c) an affected patient bearing the ΔF508 mutation. The results are presented in figure 17B. All things being equal and the three assays being run simultaneously in parallel, figure 17B shows that, this time, the maximum signal is collected from the sample provided by the affected patient indicating that both DNA genes annealed with the ΔF508 labeled PNA probes and permitting a positive identification of the presence of mutated ΔF508 from the patient's sample; a less intense signal is collected from the sample provided by the unaffected carrier indicating that one of the genes annealed to the ΔF508 labeled PNA probe while the second gene did not and also permitting a positive identification of the presence of a mutated ΔF508 gene; no significant signal is collected from the sample provided by the unaffected individual indicating that no mutated ΔF508 genes were available from the sample to anneal with the ΔF508 labeled PNA probe.

The results of these two assays are presented in Table 3 showing the pattern of signal response from the various DNA samples with the two types of PNA probe.

**Table 3:**

| ΔF508 Mutation Detection | | | |
|---|---|---|---|
| PNA Probes | Normal DNA (Wild Type) | Unaffected Carrier (Heterzygotes) | Patient (Homozygotes) |
| Normal PNA Probe | ++ | + | -- |
| Mutant PNA Probe | -- | + | ++ |

| | | | |
|---|---|---|---|
| --: No PNA/DNA hybrid peak found | | | |
| +: The magnitude of the hybrid peak is one +. | | | |
| ++: The magnitude of the hybrid peak is twice than +. | | | |

### Example 8 The Cystic Fibrosis W1282 Gene

### A. Experimental Design

A similar experiment as above. was conducted for the Cystic Fibrosis gene W1282. The Cystic Fibrosis gene W1282 has a single point mutation. The experimental procedure is describe above in Example 5. The following PNA probes for the wild type (W1282) and the mutant gene (W1282X) were prepared:

W1282 (WT probe) Flu-oo-CTTTCCTCCA CTGTT-NH₂

W1282X (M probe) Flu-oo-CTTTCCTTCA CTGTT-NH2

### B. Results

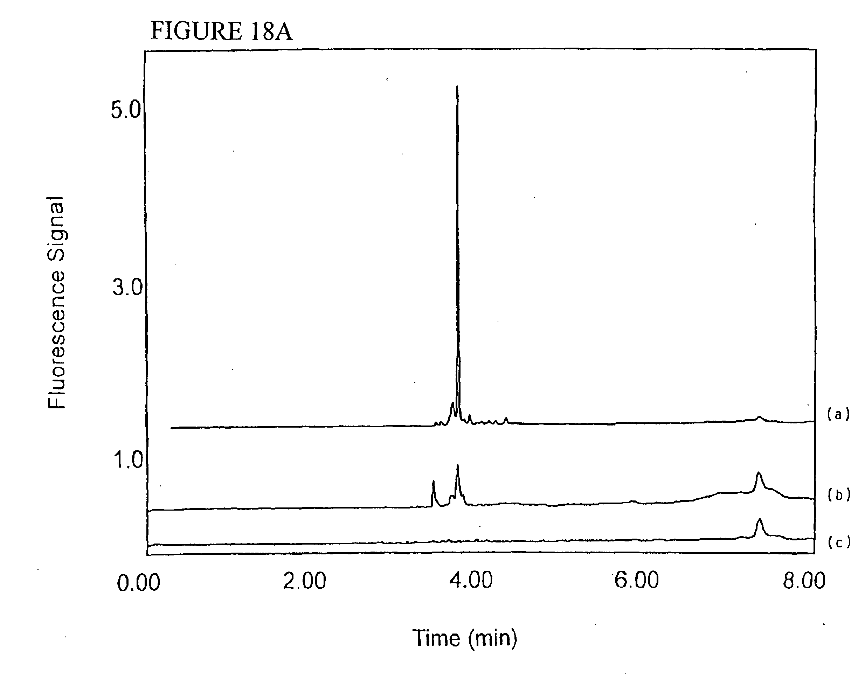

A hybridization assay for the wild type Cystic Fibrosis W1282 gene was conducted by capillary electrophoresis with the two labeled PNA probes: (a) the WT probe and (b) the M probe (c) control without DNA sample but with M probe only. The results are presented in figure 18A. All things being equal, and the three assays being run simultaneously in parallel, figure 18A shows that in line (a) a strong signal is detected when using the WT probe indicating that both genes annealed to the wild type labeled PNA probe; no significant signals were detected when using the M probe indicating that the M probe did not anneal to the wild type gene in line (b) even with only one base mismatch and under the capillary electrophoresis conditons and no significant signal were detected in the control run (c).

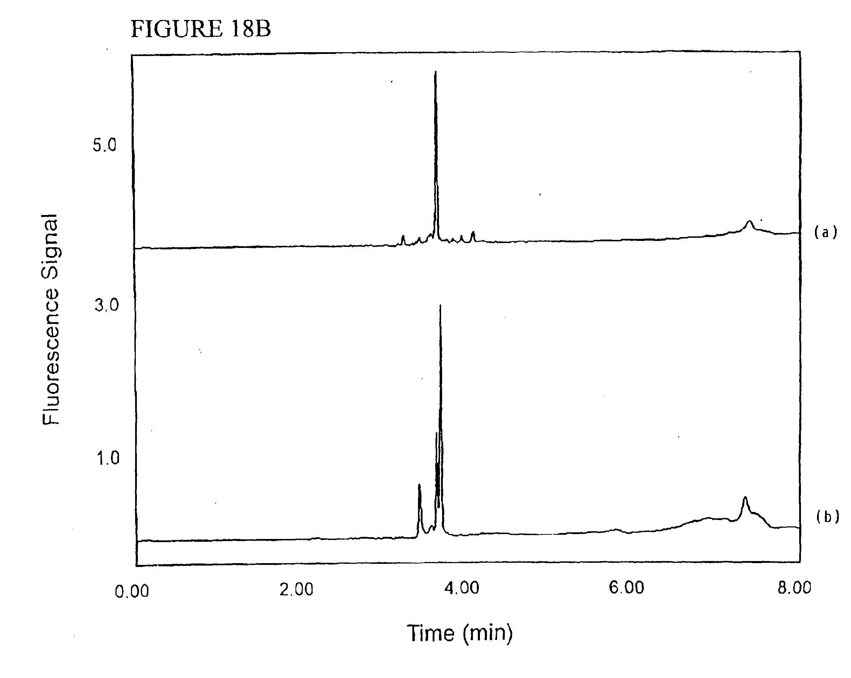

A second hybridization assay for the wild type Cystic Fibrosis W1282 gene was conducted by capillary electrophoresis with the two labeled PNA probes: (a) the WT probe and (b) the M probe. The results are presented in figure 18B. All things being equal and the three assays being run simultaneously in parallel, figure 18B shows that, this time, a relatively less intense signal but nonetheless strong signal is collected when using either WT or M probes indicating that both probes anneal to the sample DNA provided by a carrier permitting a positive identification of the presence of a mutated W1282X gene in the sample. The results of these two assays are presented in Table 4 showing the pattern of signal response from the various DNA samples with the two types of PNA probe.

**Table 4:**

| W1282 Mutation Detection | | |
|---|---|---|
| PNA Probes | Normal DNA (Wild Type) | Healthy Carrier (Heterzygotes) |
| Normal PNA Probe | ++ | + |
| Mutant PNA Probe | -- | + |

| | | |
|---|---|---|
| --: No PNA/DNA hybrid peak found | | |
| +: The magnitude of the hybrid peak is one +. | | |
| ++:The magnitude of the hybrid peak is twice than +. | | |

### Example 9 Multi-PNA Probes for Multi-Gene Detection

### A. Experimental Design

The CF w1282 gene (wild type PCR product) and CF F508 gene (wild type PCR product) were used as a model system. Fluorescein labeled normal PNA probes for normal w1282 gene and normal F508 gene were added to the mixture of the two genes. After hybridization, the mixture was injected into the HEC (Hydroxyethylcellulose) gel-filled capillary. The capillary's length was 27 cm total with 20 cm of separation length to the detector.The internal diameter of the uncoated capillary was 75 µm. Separation was performed with presence of 2M urea in TBE (Tris-Borate-EDTA) buffer. Since the two genes differ in size (197 bp for CF w1282 and 384 bp for CF F508), the separation of the hybrids was completed in 5 min.

### B. Results

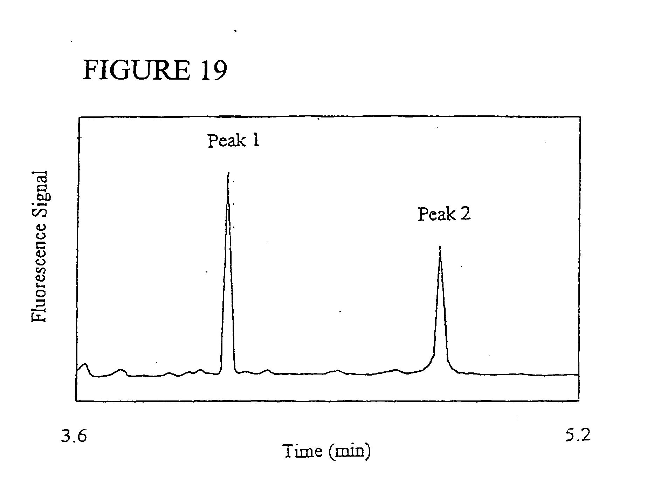

Referring to figure 19, the duplex with the shorter gene CF W1282/PNA is detected first and is shown as Peak 1, the duplex with the longer gene F508/PNA is detected second and is shown as Peak 2.

This experiment shows that multi-PNA probes for multi-genes probing in CE can be extended to multi-PNA probes for multi-gene probing. Furthemore, PNA probes tagged with different color or enzyme labels could be used to detect different mutations of the same gene or mutations of different genes simultaneously in the same analysis.

It will be apparent to those skilled in the art that various modifications and variations can be made in the methods, kits and apparatus of the present invention without departing from the spirit or scope of the invention. Thus, it is intended that the present invention cover the modification sand variations of this invention provided they come within the scope of the appended claims and their equivalents.

## Claims

1. An apparatus for detecting at least one selected target sequence in at least one polynucleotide, said apparatus comprising:
a) a sample introduction zone;
b) at least one PNA probe, disposed to mix with a sample comprising at least one double stranded polynucleotide introduced to said introduction zone, said at least one PNA probe having a sequence complementary to said selected target sequence suspected to be present in said at least one double stranded polynucleotide;
c) a nucleic acid/nucleic acid denaturing reagent disposed to permit the formation of a PNA/nucleic acid complex when said selected target sequence is present, said formation occurring under denaturing conditions in the presence of said at least one nucleic acid/nucleic acid denaturing reagent; and
d) a separation zone, optionally a capillary channel, in communication with said introduction zone, said separation zone separating said PNA/nucleic acid complexes from other components present in said introduction zone and said separation zone under denaturing conditions; and optionally further comprising a detection zone in communication with said separation zone, and/or a sample incubation zone disposed in communication with the sample introduction zone and in communication with the separation zone.

2. A microchip apparatus comprising a plurality of capillary channels, each said capillary channel further comprising:
a) a sample introduction zone;
b) at least one PNA probe, disposed to mix with a sample introduced in each said introduction zone, said sample comprising at least one double stranded polynucleotide and said at least one PNA probe having a sequence complementary to a selected target sequence suspected to be present in said at least one double stranded polynucleotide;
c) a nucleic acid/nucleic acid denaturing reagent permitting the formation of a PNA/ nucleic acid complex when said selected target sequence is present, said formation occurring under denaturing conditions in the presence of said at least one nucleic acid/nucleic acid denaturing reagent;
d) a detection zone; and
e) a separation zone in communication with said introduction zone and said detection zone, said formation occurring under denaturing conditions in the presence of said at least one nucleic acid/nucleic acid denaturing reagent; and optionally each said capillary channel further comprising disposed in communication with said sample incubation zone and said separation zone.

3. The apparatus of any one of claims 1 to 2, wherein the separation zone comprises a sieving medium, and optionally the sieving medium is selected from the group consisting of polyacrylamide, agarose, polyethylene oxide, polyvinyl pyrolidine and methylcellulose.

4. The apparatus according to any one of claims 1 to 2, wherein the at least one PNA probe comprises a charge-modifying moiety, or is associated with a particle, or is labelled with a detectable moiety, and optionally the detectable moiety is selected from the group consisting of enzymes, colored particles, fluorophores, biotin, chromophores, radioisotopes, electrochemicals and chemiluminescent moieties.

5. The apparatus according to any one of the claims 1 to 2, wherein the at least one PNA probe is labelled with a detectable moiety selected from the group consisting of enzymes, colored particles, fluorophores, biotin, chromophores, radioisotopes, electrochemical and chemiluminescent moieties.

6. The apparatus according to any one of the claims 1 to 2, wherein the denaturing reagent is selected from the group consisting of: urea; formamide; organic solvents; a low ionic strength buffer that permit adjustment of the mixture resulting from formation of a PNA/nucleic acid complex to low salt concentrations wherein the temperature of the mixture is adjusted to render the medium denaturing.

7. The microchip apparatus of claim 2, wherein the separation zone of at least one of said capillary channel comprises a sieving medium, and optionally the sieving medium is selected from the group consisting of polyacrylamide, agarose, polyethylene oxide, polyvinyl pyrolidine and methycellulose.

8. A method for detecting a plurality of selected target sequences in polynucleotides, said method comprising the steps of:
a) providing a sample comprising at least one single stranded nucleic acid sequence and its complementary strand, wherein said at least one single stranded nucleic acid sequence and its complementary strand is suspected to include a plurality of selected target sequences;
b) mixing said sample with a plurality of PNA probes each having a sequence complementary to at least a portion of a respective one of said selected target sequences of said at least one single stranded nucleic acid sequence and its complementary strand under conditions permitting the formation of at least one PNA/nucleic acid complex when said respective one of said selected target sequences is present, said formation occurring under denaturing conditions in the presence of said at least one nucleic acid/nucleic acid denaturing reagent;
c) separating said at least one PNA/nucleic acid complex from other components of the mixture from step b) under denaturing conditions; and
d) detecting said at least one PNA/nucleic acid complex; or optionally the method of claim 1 wherein step b) comprises mixing said sample with a plurality of PNA probes, each having a sequence complementary to at least a portion of a respective selected target sequence of one of said at least one strand under conditions permitting the formation of at least one PNA/nucleic acid complex when said respective selected target sequence is present.

9. A method for detecting a selected target sequence in a mixed sample solution of single stranded nucleic acids, said method comprising the steps of:
a) mixing the sample with a PNA probe having a sequence complementary to at least a portion of said target sequence, if present, under denaturing conditions thereby to form a detectable PNA/nucleic acid duplex;
b) after step a) separating the components in the sample under denaturing conditions;
c) detecting said duplex.

10. The method of claim 9 wherein step b) is performed in a denaturing medium.

11. The method of claim 10 wherein the denaturing medium is a sieving medium.

12. The method of claim 9 wherein the PNA probe is labelled.

13. The method of claim 11 wherein the sieving medium is selected from the group consisting of polyacrylamide, agarose, polyethylene oxide, polyvinyl pyrolidine and methylcellulose.

14. The method of claim 12 wherein the label is selected from the group consisting of enzymes, fluorophores, biotin, chromophores, radioisotopes, colored paricles, electrochemical and chemiluminescent moieties.

15. The method of claim 9 wherein the species are separated electrophoretically.

16. The method of claim 15 wherein the species are separated by capillary electrophoresis.

17. The method of claim 9 wherein the denaturing medium comprises a denaturing reagent.

18. The method of claim 9 wherein the medium is rendered denaturing by adjusting the temperature of the medium.

19. A kit for the separation of the components of a mixed sample solution of single stranded nucleic acids and their complementary strands, and for detecting therein a selected target sequence, comprising
a) a detectable PNA probe having a sequence complementary to at least a portion of said target sequence in an electrophoretic medium, and
b) a denaturing sieving medium.

20. The kit of claim 10 wherein the electrophoretic medium is disposed in a capillary or channel.

21. The kit of claim 20 comprising at least two PNA probes, each having a sequence complementary to a different said target sequence.
